# EUROPEAN PATENT SPECIFICATION

(11) **EP 3 440 618 B1**
(45) Date of publication and mention of the grant of the patent: **20.11.2024**
(21) Application number: 17779979.8
(22) Date of filing: 10.04.2017
(51) Int. Cl.: G06Q 10/06, G06Q 50/22

(54) **VIDEO-BASED ASYNCHRONOUS APPOINTMENTS FOR SECURING MEDICATION ADHERENCE**
VIDEOBASIERTE ASYNCHRONE TERMINE ZUR SICHERUNG DER ADHÄRENZ BEI MEDIKAMENTEN
RENDEZ-VOUS VIDÉO ASYNCHRONES POUR LA SÉCURISATION D'UN RESPECT DE PRISE DE MÉDICAMENT

(30) Priority: 08.04.2016 US 201662320363 P
(43) Date of publication of application: 13.02.2019
(73) Proprietor: Emocha Mobile Health Inc., Baltimore, MD 21205 (US); The Johns Hopkins University, Baltimore, MD 21218 (US); Shah, Maunank, Baltimore, MD 21218 (US); Bollinger, Bob, Baltimore, MD 21218 (US); Chang, Larry, Baltimore, MD 21218 (US); McKenzie-White, Jane, Baltimore, MD 21218 (US)
(72) Inventor: SHAH, Maunank, Baltimore, MD 21218 (US); BOLLINGER, Bob, Baltimore, MD 21218 (US); CHANG, Larry, Baltimore, MD 21218 (US); MCKENZIE-WHITE, Jane, Baltimore, MD 21218 (US); SEIGUER, Sebastian, Baltimore, MD 21205 (US)
(74) Representative: Grünecker Patent- und Rechtsanwälte PartG mbB
(86) International application number: PCT/US2017/026851
(87) International publication number: WO 2017/177235

(56) References cited:
- WO-A1-2015/059306
- US-A1- 2011 275 051
- US-A1- 2012 041 788
- US-A1- 2012 316 897
- US-A1- 2014 055 589
- US-A1- 2015 281 949

## Description

### BACKGROUND OF THE INVENTION

The present disclosure relates in general to health care, and in particular to systems and methods for video Directly Observed Therapy (DOT).

Medication adherence is a significant problem in the United States, with adherence levels for self-administered medication at about 60%. Tuberculosis (TB) is contagious and has a high mortality rate, which is why the long-held standard of care is Directly Observed Therapy, or DOT. In DOT, the clinician is required to watch the patient take their medication in person. With DOT, adherence levels of over 90% can be achieved, but requires daily, in-person treatment, which can be far too costly for the system and burdensome for the patient. Other medications may be administered using DOT, such as Hepatitis C Virus (HCV) medications, HIV medications, opioid addition therapy medications, etc.

In the United States, health departments spend millions of dollars driving to patients' homes every day to conduct DOT on TB patients. While there are only approximately 10,000 new cases of TB per year, the high mortality rate makes DOT critical. As such, health departments use their limited resources in such a manner because poor adherence to TB treatments can lead to more dangerous drug resistant forms of TB, spread of the disease, and deterioration of the patient's health. More than 11 million people in the U.S. have Latent TB Infection (LTBI). In many cases, LTBI is managed by public health departments. The most effective regimen for LTBI is a 12-dose, 12-week regimen. Directly Observed Therapy (DOT) is the CDC recommended standard of care. However, other self-administered regimens are often prescribed due to lack of public health department resources to perform DOT. Better methods of easing the burden of DOT-based medication regimens are needed.

With advances in computer technology and the Internet, home verification systems have been devised. For example, U.S. Pub. 20160259911 "Automated Medication Adherence System" describes the use of real time video to verify a patient taking medication: "In application, the patient faces the video camera so that the operator at the central computer can witness the patient administering the medication. Further, during the entire administering session, neither the medication container nor the dosage is ever removed from the view of the camera and the operator stationed at the central computer so that while the prescribed medication is being administered by the patient, the medication remains in front of the camera until completely administered by the patient." (Abstract).

U.S. Pub. 20080076973 "Remote Health Care System With Treatment Verification" describes a similar system: "The patient presents the pill to the video camera 102 such that the medical professional is able to confidently identify it. The patient is then instructed to ingest the pill. The patient ingests the pill. The medical professional then optionally requests that the patient open their mouth to clearly show that the pill is not in their mouth. Further optionally, the patient shows the hand that held the pill before the pill was ingested. In this way the medical professional is able to confidently establish that the patient has swallowed the pill." (paragraph 0028).

U.S. Pub. 20150061832 "Directly Observed Thearpy [sic] Using Mobile Technology" describes using video in combination with unlocking a compartment of a medication dispensing device: "The device may be used to acquire video data showing the identity of the patient, and to transmit that data to a first computing device at a control center for verification of the patient's identity, as described above. After the patient's identity has been verified, the device may receive a signal from the control center causing the solenoid to rotate the dispenser until a chamber (1-7) is positioned adjacent the retractable window. Medication within that chamber can then be removed by the patient. An application running on the device subsequently may cause the touch screen to display instructions for acquiring video of the administration or ingestion of the medication, and subsequently transmitting that video to the control center to verify that the patient has complied with their therapy." (paragraph 0026).

While the above described systems eliminate the need for travel to monitor a patient taking medication, it would be desirable to further limit the burden on medical personnel.

US 2014/055589 A1 discloses the asynchronous remote monitoring of a patient taking medication but fails to secure the recording against manipulations.

### SUMMARY

The present invention provides a system and method for asynchronous, remote monitoring of a patient taking medication that satisfies the requirements for Directly Observed Therapy ("DOT"). The present invention also provides a technological triage system for remotely monitoring and intervening with DOT patients using an Internet based DOT program that insures patients most needing attention are addressed first.

In one embodiment, patient dosage data and a corresponding medication regimen are stored in a patient profile. A patient securely accesses the profile and is prompted to record a video file showing the patient taking the medication according to the regimen. The video file is encrypted and uploaded to a secure database. A medical provider is notified of video files awaiting review, prioritized according to a triage method that ranks review urgency based on a patient's demonstrated regimen adherence history. The medical provider securely accesses the video file to verify adherence, and indicates acceptance or rejection of the video file as proof of the patient taking a correct dosage of the medication as defined in the medication dosage regimen. A medication regimen adherence value is calculated based on the medication dosage regimen and the approval data. The degree of monitoring of the medication dosage regimen by the medical provider is modified based on the medication regimen adherence value.

In one embodiment, a patient is progressed through multiple stages based on whether or not they submit compliant videos. As the patient demonstrates continuing adherence to the protocol, the time period within which the medical provider is required to review the video is relaxed (e.g., from 6 hours to 24 hours to 48 hours). Also, the intensity of intervention is relaxed with demonstrated adherence, such as not requiring a phone call to the patient for a single missed video or non-compliant video.

In one embodiment, a patient progresses through at least 3 stages. The first stage, App Download, requires the patient to successfully download the application and record and upload a test video. If the patient fails after multiple tries, they become ineligible for the Internet based DOT program. Upon successful completion of the App Download stage, the patient enters an Onboarding stage with high intervention intensity. The patient is required to demonstrate 100% compliance of at least two video recordings to move onto the third stage. In the third stage, less than 100% compliance is required, and the intervention intensity is reduced after a period of demonstrated compliance. The intervention intensity is increased if there is a faltering in the demonstrated compliance. In one embodiment, the third stage is itself composed of 3 stages, the Habit Building stage, Performance stage and Autopilot stage, with different degrees of required compliance and intervention intensity.

### BRIEF DESCRIPTION OF THE DRAWINGS

FIG. 1 shows a simplified method 100 for enabling asynchronous appointments for securing medication adherence, according to certain embodiments.
FIG. 2 is a simplified block diagram of a security architecture according to certain embodiments.
FIG. 3 is a block diagram of a program architecture according to certain embodiments.
FIG. 4 is a block diagram of an infrastructure architecture according to certain embodiments.
FIG. 5 is a table of the various intervention intensities and compliance criteria according to certain embodiments.
FIG. 6 is a table of the requirements for various stages for a 5-7 day regimen according to certain embodiments.
FIG. 7 is a table of the requirements for various stages for a 3 day regimen according to certain embodiments.
FIG. 8 is a table of the requirements for various stages for a 1 day regimen according to certain embodiments.
FIGS. 9A-B are a high level flow chart of the stages according to certain embodiments.
FIG. 10 is a more detailed flow chart of one of the stages according to certain embodiments.
FIG. 11 is a diagram of a medical provider triaged review interface according to certain embodiments.
FIG. 12 is a simplified block diagram of a computing system and client computing system according to an embodiment.

### DETAILED DESCRIPTION

In the following description, various embodiments of systems and methods for providing mobile-based, computer-aided medical care will be described. For purposes of explanation, specific configurations and details are set forth in order to provide a thorough understanding of the embodiments. However, it will also be apparent to one skilled in the art that the embodiments may be practiced without the specific details. Furthermore, well-known features may be omitted or simplified in order not to obscure the embodiment being described. For example, although a mobile internet-based Directly Observed Therapy platform is described, access need not be through a mobile device, and could use a desktop computer, smart television, game console, or any other computing device with a camera, or connectable to a camera or media from a camera.

In certain embodiments, asynchronous appointments may be used to secure medication adherence for patients requiring directly observed care. Using asynchronous video DOT, asynchronous video-based direct observation allows for the patient and the healthcare worker to engage in the activities of a face-to-face appointment with each party performing their aspect of the appointment at different times, independent of one another. In some embodiments, the purpose of such appointments can be for the healthcare worker to verify ingestion, inhalation, or infusion of medication through observation. While a live, in-person appointment may be replicated nearly exactly through a live video observation, asynchronous video DOT enables a non-live appointment, which would not be possible without the use of the technology. The patient is free to take their medication at any time during a "check in window," while the healthcare worker is able to assess the video with the assistance of the technology at any time thereafter.

In some embodiments, many different permutations of asynchronous appointments can be supported. For example, a patient may be required to take their medication (if there are multiple pills) at many times throughout the day. The healthcare worker can assess the entire episode at a later time, reviewing each video separately or as single compiled video (referred to as "batching"), and assess all of them together as one episode or "dose." Medication adherence can then be calculated accordingly. In another scenario, a patient can take their medication at one or more times throughout the day, and several healthcare workers can review and accept the dosage, as if in-person, each at different times (i.e., asynchronous direct observed care) in the day.

In a typical operation, a patient can record a video of themselves taking their prescribed medication via asynchronous video DOT software on their mobile computing device (e.g., smart phone, laptop computer, smart watch, etc.). If the patient has internet access, the recorded videos can be sent immediately to the asynchronous video DOT server. The video files can be sent individually or may be batched, as noted above. In situations where internet access is sparse or intermittent, or in areas with very slow data rates, portions of the individual videos may be sent, whenever possible, from the patients computing device and may be reassembled at the asynchronous video DOT server side.

In situations where a patient may have extended periods of time with no internet access, the patient can operate their asynchronous video DOT software offline and continue to take their prescribed mediation(s) and record a video. The videos will continue to be stored on the patient's mobile computing device until an internet connection becomes available. The videos may be stored in any suitable format on the patient's computing device, and may be saved individually or batched, and individual video files may be saved as a number of pieces or portions of the entire video entry (in consecutive or non-consecutive order) and reassembled at a later time by the asynchronous video DOT server(s) when transferred. Once their computing device establishes an internet connection, the stored videos can be sent to the asynchronous video DOT server for medical provider access. In that way, the medical provider may than review all of the videos at once and approve or disapprove each video in their own time - asynchronously with the actual time that the medication was administered, but still within the guidelines of typical programs requiring DOT-based verification.

In certain embodiments, each recorded video may be encrypted to prevent unintended parties from accessing and viewing them. The videos can be encrypted while on the patient's mobile computing device, while they are transmitted from the mobile device to the asynchronous video DOT server computer(s), and while saved on the asynchronous video DOT server computer(s). Different types of encryption may be used or combined. For instance, the video files may be encrypted on patient's mobile device and further encrypted during transmission to the asynchronous video DOT server(s). In some embodiments, the video recordings are not accessible while they are stored on the patient's mobile phone. For example, the patient cannot access the videos on their mobile device using their standard operating system media library that may store other photos, videos, audio recordings, and the like. If a user accesses or copies the files using directory management software (e.g., Windows Explorer), the files can still be encrypted to prevent access to unauthorized viewers.

In certain embodiments, a dose-by-dose adherence calculation using asynchronous direct observation can be performed with specialized asynchronous video DOT-based software. The software provides workflow and visualization to bring the two independently-performed activities together in such a way as to allow for an extremely accurate, dose-by-dose calculation of the medication adherence rate. This calculation would not be possible for patients who are not under direct observation for every single dose. As such, medication adherence as a dose-by-dose statistic is not currently available in medical care without direct observation. The implementation of asynchronous video DOT can solve this problem.

Conventional methods may use other calculations currently used as proxies for the actual dose-by-dose adherence rate. For example, a currently accepted medical standard is called Proportion of Days Covered and calculates medication adherence based on the timeliness of the patient picking up their medication from the pharmacy. There are a few exceptions, such as Tuberculosis (TB), where the standard of care is direct observation of ingestion. In TB care, the patient travels to the clinic, or a healthcare worker travels to the patient, nearly every day. The healthcare worker verifies several aspects of care, as required by the CDC: 1) identification of the medication; 2) capture of any side effects experienced by the patient since the last dose; 3) medication ingestion; and 4) document the visit.

It should be noted that the video-based asynchronous appointments for securing medication adherence, as described herein, can be applied to any suitable medication. For instance, applications of the embodiments described herein can work equally well for infections like TB, chronic conditions like Hepatitis C or congestive heart failure, or other conditions like hemophilia, asthma, and more, as would be appreciated by one of ordinary skill in the art. Also, multiple prescriptions can be applied to the various embodiments presented in this disclosure. For instance, asynchronous video DOT software can manage the administering of multiple medications in separate patient-recorded video files, or in the same video file (e.g., the patient records taking a dose of two medications at the same time on the same video).

In some embodiments, an assessment of video(s) to calculate adherence using asynchronous video DOT can include the patient submitting a video of themselves, which includes sound and images. Unlike a "smart pill" bottle, which may provide information that the medicine bottle was opened, the adherence calculation is driven by both a video being submitted and the healthcare worker(s) reviewing the video and determining that the medication was taken. By the healthcare worker(s) accepting or acknowledging that a prescribed dose of medication was properly taken, based on the video(s), the adherence calculation is increased by a dose. By rejecting or denying the fact that a prescribed dose of medication was taken, the number of doses taken does not increase, but the healthcare worker may fill out a report explaining the rejection.

In some implementations, asynchronous video DOT software can capture visual evidence that medication was taken, or can be used to infer that medication was "missed" or not taken. When a dose is not taken, the patient's regimen can be automatically extended by one day into the future. Side effects can be captured and reported through a questionnaire via a asynchronous video DOT software interface. If the patient or the healthcare worker chooses, the side effects as well as all application instructions can be displayed in another language, or as icons/images for those who cannot read one of the languages available. In some embodiments, asynchronous video DOT software can generate notifications and sent using any suitable communication protocol including SMS, email, or in-application notifications. The asynchronous video DOT software may provide notifications that can be directed to the patient or the healthcare worker and may inform of side effect reports, missed doses, or patients determined to be at risk of missing a dose in the near future.

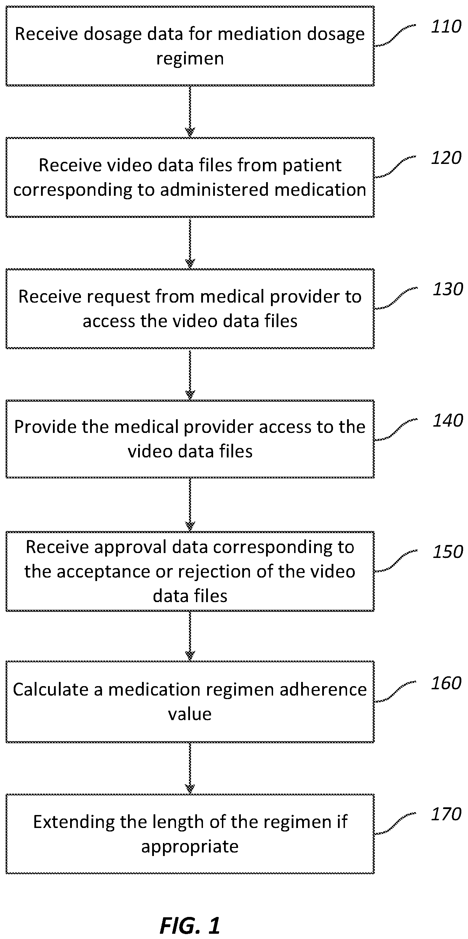

FIG. 1 shows a simplified method 100 for enabling asynchronous appointments for securing medication adherence, according to certain embodiments. Method 100 can be performed by processing logic that may comprise hardware (circuitry, dedicated logic, etc.), software operating on appropriate hardware (such as a general purpose computing system or a dedicated machine), firmware (embedded software), or any combination thereof. In certain embodiments, method 100 can be performed at least by the systems shown and described in the appendices.

At step 110, method 100 includes receiving, by a processor, dosage data corresponding to a medication dosage regimen for a medication prescribed to a patient. The dosage data may include a type of medication to be administered, a frequency that the medication is to be administered, and a dosage that the medication is to be administered.

At step 120, method 100 includes receiving, by the processor from a remote mobile device, one or more video data files including video of the patient administering the medication at intervals defined by the medication dosage regimen. The remote mobile device can be one of a smart phone, personal digital assistant, a smart watch, a smart headset, a laptop computer, a desktop computer, or a tablet computer. The one or more video data files each include a corresponding date and time stamp.

At step 130, method 100 includes receiving a request from a medical provider to access the one or more video data files. In some implementations, a request for video data files may require the medical provider to provide credentials to prove that they are an approved medical provider that can access the video data files. At step 140, method 100 includes providing the medical provider access to the one or more video data files.

In some embodiments, a medical provider can be a primary care provider or staff thereof, or may be a referred care provider (i.e., third party). asynchronous video DOT can be configured to allow any approved medical provider or individual access to view and/or evaluate (accept/reject) a patient's video data files.

At step 150, method 100 includes receiving approval data corresponding to the medical provider's acceptance or rejection of the one or more video data files as proof of the patient taking a correct dosage of the medication as defined in the medication dosage regimen.

At step 160, method 100 includes calculating a medication regimen adherence value based on the medication dosage regimen and the approval data, and, if necessary, extending the length of the regimen if appropriate (e.g., extending by a day, week, etc. depending on how many doses were missed) (step 170).

It should be appreciated that the specific steps illustrated in FIG. 1 provides a particular method 100 for enabling asynchronous appointments for securing medication adherence, according to certain embodiments. Other sequences of steps may also be performed according to alternative embodiments. For example, alternative embodiments may perform the steps outlined above in a different order. Moreover, the individual steps illustrated in FIG. 1 may include multiple sub-steps that may be performed in various sequences as appropriate to the individual step. Furthermore, additional steps may be added or removed depending on the particular applications. One of ordinary skill in the art would recognize and appreciate many variations, modifications, and alternatives of the method 100.

### Security Architecture

The asynchronous video DOT platform is a suite of patient engagement and medication adherence applications, with both mobile (iOS / Android) and web components. Health Insurance Portability and Accountability Act of 1996 (HIPAA) is the United States of America legislation that dictates the rules and provisions around data security and privacy to safeguard medical information. The asynchronous video DOT platform complies with HIPAA regulations on how to handle Protected Health Information (PHI), including but not limited to secure encryption of data, access controls, and industry-standard best practices. HIPAA has specific rules around how data should be handled and secured; for the purposes of the Cloud, the following apply:

### HIPAA Privacy Rule

HIPAA Privacy rule defines Protected Health Information as any information that can identify a patient:
- Name, Address, Birth Date, Social Security Number
- An individual's health or mental condition
- Any care provided to the individual
- Information concerning payment for services

### ADMINISTRATIVE REQUIREMENTS TO SATISFY THE HIPAA PRIVACY RULE INCLUDE:

- Assignment of a privacy official
- Employee training
- Technical and Physical Safeguards
- Process for complaints
- If PHI is disclosed, mitigation

### HIPAA Security Rule

HIPAA security rule requires covered entities to maintain reasonable and appropriate administrative, technical and physical safeguards to protect PHI. Specifically,
- Ensure the confidentially, integrity and availability of all PHI created, received, maintained and transmitted
- Identify and protect against reasonably anticipated threats to the security and integrity of information
- Protect against reasonably anticipated, impressible uses or disclosures
- Ensure compliance by workforce

### HIPAA enforcement

Technical and Physical Safeguards used for the present invention are listed below:
- Data is stored encrypted-at-rest
- Data is encrypted-in-flight
- All events are audit-logged
- All system access and keys are safeguarded and established according to a process
- All systems are highly-available and have disaster recovery protocols
- All system configurations are documented
- All data access is on a need-to-know basis
- Technical safeguards are in-place (such as anti-virus, anti-malware)
- Patients have the right to access their data at any time

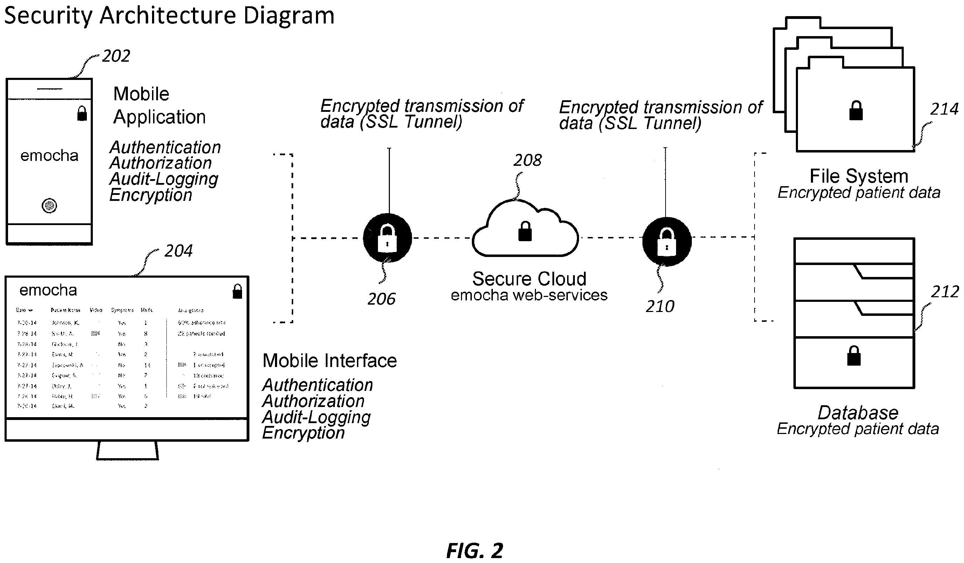

FIG. 2 is a simplified block diagram of a security architecture according to certain embodiments. A user device 202 has an application loaded for secure access with a password and encryption capability. A web interface device 204 for a medical provider similarly has an application with secure access capability. Both user device 202 and web interface device 204 use an SSL tunnel, such as SSL tunnel 206, to communicate over the Internet 208 and through SSL tunnel 210 to a secure database 212 and file system 214.

### Access

In one embodiment, access to the system is managed via password-protected user accounts. User passwords are never stored in clear text, only as a one-way encrypted digest, and are never visible to any user including asynchronous video DOT system administrators. The system includes rules to require that users create a complex password (with configurable minimum length, and requirements for special characters, numbers, etc.), and requires that user passwords be changed periodically. All access attempts, successful or otherwise, are logged. Repeated failed attempts result in the account being locked, and may only be unlocked by an administrator.

### User roles and administration

In one embodiment, user accounts are defined by a set of role-based permissions and only users with elevated permissions are capable of modifying a user's access. The system's position towards any user action is "default-deny" - unless a user has specifically been granted the right to perform an action via a permission they've been explicitly granted by an administrator, the action is not permitted. An interface is provided both to add and remove roles from user accounts, to define new roles, and to add and remove permissions from existing roles. Multiple roles can be assigned to the same user.

### Input validation

In one embodiment, all input to the system is checked for validity before being processed into data structures. Validation is done on both the client and server side; client-side as a user-experience convenience and data input validation, and server-side for data validation. The data validation processes are required to run all inputs before any data is stored. The validation involves but is not limited to formatting, length, range, data type validations, along with general malicious attack checks such as Cross-Site Scripting (XSS) attacks and Structured Query Language (SQL) injection attacks. If any validation fails, the whole request is declined and data is not stored.

### Authentication & Authorization

In one embodiment, authentication is managed with a username and password. Users are authorized to perform only the actions explicitly granted to them by the roles they have been assigned. Before allowing any user interaction with the system (for instance viewing patient information, creating a new user account, scheduling an appointment), the user's permissions are checked to determine if they have the privileges to perform the specified action and whether they're allowed to access the object in question. A given user may be configured to only be allowed access to view or modify certain patients. In the event that a user has forgotten their password, they can request a password reset for their username. This will generate a message to the email address associated with that account containing a time-limited link which can be used to enter a new password.

### Session Management & Timeouts

In one embodiment, most interactions within the system require a valid, recent, session token which is returned as part of a successful authentication. Session identifiers are stored as encrypted cookies on the device or browser and chosen from a large, random, address space. These are not predictable and any modification of the local value will invalidate the session. No user-provided data (other cookies, roles/permissions, etc.) is used by the system and is ignored if provided. The platform only trusts the platform's database as the trusted source of information for authentication and session management. Sessions time out after a configurable period of inactivity, for which the default is 5 minutes. When timeout happens, the user must re-authenticate to continue interacting with the system.

### Encryption

In one embodiment, the asynchronous video DOT platform uses two main kinds of encryption: in-flight and at-rest. In-flight encryption refers to the encryption of all data while being transmitted. Data being sent from a client, whether web-based or a mobile application, is sent over a secure HTTPS connection secured by a 2048-bit Transport Layer Security (TLS) certificate. The system audits the TLS configuration regularly, ensuring that system configuration is as up-to-date as possible. All connections between the database and application servers are made over TLS as well, utilizing the same 2048-bit certificate.

At-rest encryption means that all Protected Health Information (PHI) in the database and disk is always stored encrypted. This includes any record of a user, anything in the error log or audit log tables, any patient data, and all information submitted including video files or GPS coordinates. The encryption scheme uses the Advanced Encryption Standard (AES) algorithm of at least 256 bits, with the ability to revoke and issue new keys as needed.

Key Management for the encryption / decryption keys is accomplished through a separate server and only accessible through a highly-restrictive API through defined methodologies. Keys are only stored in-memory on the application server and never in permanent files written to disk. Effectively, the database cannot decrypt its own data; even in the event of the server being compromised and a malicious party acquiring an export of the data, PHI will remain secure. Each customer application's data (patients, check-ins, laboratory test result data) resides in a separate database and is completely invisible to other customers.

### Audit logs

Any view or edit of the system configuration or data is logged in a persistent and unmodifiable database. Audit trail records include but are not limited to the action being taken, the user who initiated the action, the date and time of the action, and in the case of modifications, both the old and new values. These logs are accessible through the administrative interface.

### Infrastructure, Hosting Environment and Backups

In one embodiment, asynchronous video DOT platform's servers are hosted "in the cloud" at secure data centers. Physical access to servers are limited; all servers are single-tenant; and there is no shared hardware between asynchronous video DOT platform and any other entity. Network access to any server is limited to the specific port and IP ranges needed for the platform to function. For instance, the front-facing load balancers allow access via HTTPS from anywhere but the database servers only allow TLS access over port 3306 from the application servers they specifically support, and SSH access is only permitted from the asynchronous video DOT platform offices. Login credentials are managed by a combination of a strong authentication (username / password) and private keys. Login passwords are required to adhere to the asynchronous video DOT Password Policy, explained above. Access to production servers is only given to employees with a demonstrable need for access, mostly to provide support & maintenance for the platform.

In one embodiment, remote console access, where these settings are managed, requires a two-factor authentication token in addition to a strong password. Accounts used to connect to servers are per-user rather than a shared "root" account. This allows for individual users to be managed, or access revoked, without compromising other accounts. Backups are taken regularly; nightly backups are made of the entire encrypted database and snapshots of the entire server disk are taken, including any encrypted file uploads. These are kept available across a rolling 30-day window in case they need to be rolled back to. All data is replicated across multiple servers, in near real-time, to ensure availability. We also run monitoring software that checks, at all times, whether the service is available and functioning, and have a system in place to page on-call support personnel if needed. In addition, nothing is ever deleted in the system; data is "soft-deleted" via marking with a flag that will hide the record during normal operations, but leave it easily recoverable if needed.

### Design Overview

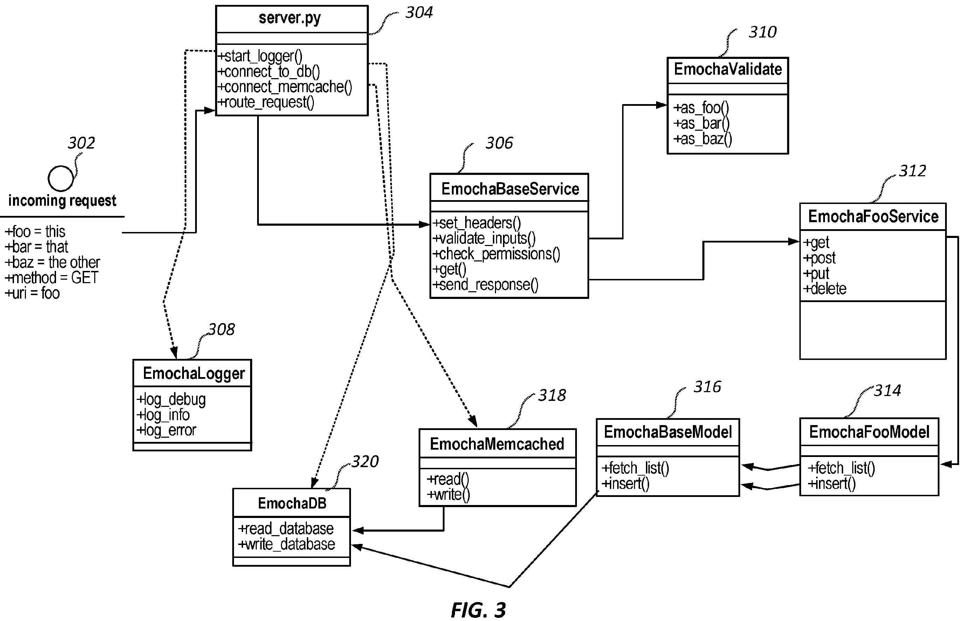

FIG. 3 is a block diagram of a program architecture according to certain embodiments. A universal RESTful API is provided, communicating entirely over HTTPS and in the JSON format (JavaScript Object Notation), and serves as the means of interaction with both the web interface and the mobile application. Full role-based user permissions are required, as is strict validation of all user input, useful error codes. Both the web interface and mobile apps handle errors dynamically.

An incoming request 302 is provided to server.py 304. The main services logic lives in server.py 304 and lib/services/base.py, and handles routing specific URLs to the right services class. Debugging, error and other information is logged by logger 308. Base service module 306 sets the headers, validates inputs, checks permissions, gets any needed data and sends a response. Validate module 310 validates messages. FooService module 312 performs specific functions as noted under control of Base Service module 306. FooService module 312 itself uses FooModel 314, BaseModel 316, and database module 320. Server.py 304 can also directly access the database using database module 320 and memory cache module 318.

Other libraries include the memcached driver, encryption wrapper (encrypted fields are defined in the model, and the field data consist of key_version, initialization vector, and encrypted value, all concatenated together), and base database connection class. The DB and memcached drivers are capable of handling multiple connections and re-connecting in the case of a temporary outage. There's a logging class, lib/logger.py, and one to send email, lib/mailer.py.

Service URLs are defined in each service class, as a list with each element consisting of a dictionary with the following keys:
- method (GET, POST, PUT, or DELETE)
- uri (regex pattern)
- required (list of field names that, if missing or mis-formatted, will result in an error)
- optional (list of field names that, if present and mis-formatted, will result in an error)
- require_auth (whether to allow un-authorized requests)

Which gets compiled into a list of usable route/method combinations in server.py, with the required/optional/require_auth values used to validate the request input.

Required and optional fields are validated by methods in lib/validate.py, as defined in "Input Validation" above, and available to the service in self.inputs. The service classes themselves descend from EmochaBaseService, and implement a subset of the following methods: get, post, put, delete (each called when the URL is reached by the relevant HTTP method).

Service methods themselves are small and often involve simple permissions checks, often secondary validation, and calling a select/update/list wrapper around a model function. In more complex cases such as transactions that update multiple tables, they can be lengthier. Each method is a self-contained piece of functionality in order to maintain logical separation of architecture.

### Infrastructure

There are two main software scripts at the core of the services layer. They may be run on the same machine, or each on their own instance type.
- server.py, which listens on ports 8001-8004, running one process per CPU core available, and serving the JSON over REST.
   ∘ Model: Database layer
   ∘ Controller: Emocha services modules, routed through Python Tornado Web Server.
   ∘ View: JSON
- frontend.py, which listens on ports 8801-8804, running one process per CPU core, and serving the HTML templates for the frontend.
   ∘ Model: Requests to the services AP.
   ∘ Controller: Two main URI handler types:
      ▪ Web Handlers: Checks the session cookie, uses the Tornado templating engine to render the HTML template, including JS blocks to handle all dynamic content.
      ▪ JSAPI Handlers: Pass-through proxy that makes requests to services, solving AJAX same-origin issues and provides an additional layer of validation.
   ∘ View: Tornado HTML templates.

Both of these processes run behind a local nginx instance, running in proxy/load-balance configuration to route traffic across processes and handle SSL termination. Nginx listens on two ports - 80 and 443. HTTPS/443 is used for all normal traffic, and the only function of the HTTP/80 listener is to redirect traffic over to the HTTPS listener.

There are also a set of worker processes that run via cron to handle scheduled jobs for a few things:
- Data interchange with third-parties, often for test result processing.
   ∘ API-based: if the third-party has an API we can use to send/receive data, these workers will encapsulate all operations against it.
   ∘ SFTP-based: via secure File Transfer Protocol (sFTP), a third party may drop a file-based data dump to be ingested by the asynchronous video DOT platform.
- Notification engine
   ∘ Set up to run as many copies as needed
   ∘ Load configuration (which messages to send, and to whom, and when - either on a schedule or event-based) from client database.
   ∘ Send messages out via SMS, email, or in-app notifications, depending on client configuration.
- Check-in processing engine
   ∘ Updates a closed/passed/timed-out (a time window that's ended) check-in window to modify the check-in status appropriately.
      ▪ Active patients have their check-ins moved to status "MISSED"
      ▪ Paused patients have their check-ins moved to status "PAUSED"

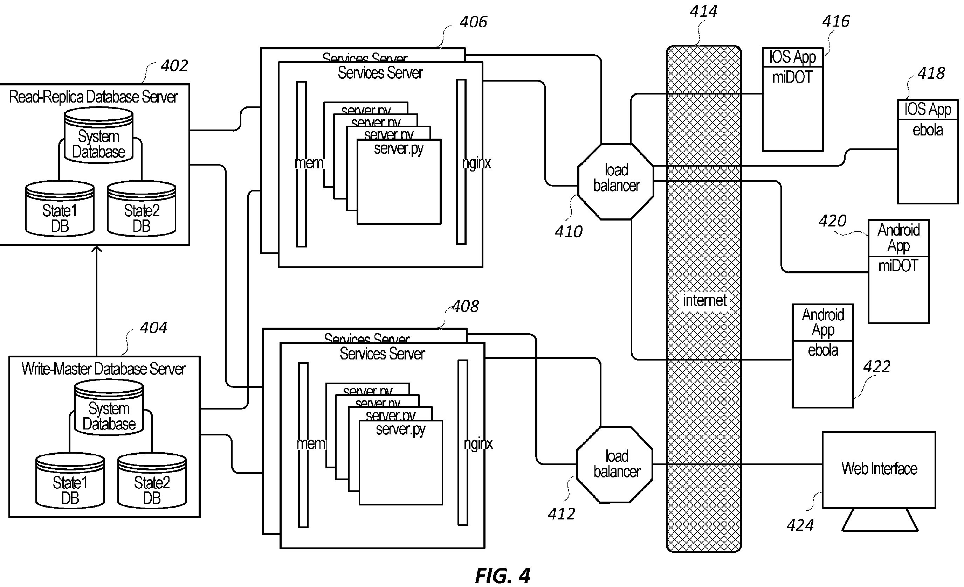

FIG. 4 is a block diagram of an infrastructure architecture according to certain embodiments. Separate database servers are provided, with a Read-replicate database server 402 and a write-master database server 404. These communicate through one or more services servers 406 and website servers 408. Load balancers 410 and 412 distribute the communications to balance the load, and are in communication, through Internet 414, with user devices 416, 418, 420, 422 and web interface 424. In one embodiment, the Services and Website load balancers 410, 412 are actually the same piece of equipment, but logically separate. In an alternate embodiment, they may run on different hardware, pointing at different clusters of app servers, to provide scalability.

### Notifications

The apps can send notifications to users/patients. A notification is defined by a few criteria:
- Who? This is a role, ebola_patient_sms, administrator sms (essentially just a role with a single permission - GET_SMS - it can't be rolled into the regular role because people might opt out).
- Where? The business unit that the role is attached to - ebola_patient_sms for _County, etc.
- What? The message content. This needs to be brief (SMS has a 160-character limit) and cannot include any identifiable information (such as patient name, medical record number, etc.).
- When or Why?
   ∘ When? Proactive messages are sent out on a schedule - eg, a daily reminder to take your pills.
   ∘ Why? Notifications may be sent based on an event - eg, a check-in with positive symptoms results in a message being sent to the healthcare workers as a notification.
- How? SMS, Email, or In-app notification.

A log is kept of all messages sent, and there is a mechanism in place to send messages that may have failed during their original sending window

### Web Applications

In one embodiment, the web application functions as a thin software layer around asynchronous video DOT platform core services - essentially there are two kinds of requests this handles. One is regular web pages, containing templated static HTMI,/CSS, with a JavaScript layer that handles all dynamic data. The second kind of "page" is the JavaScript API, essentially a proxy pass-through to a) work around AJAX Same-Origin issues, and b) hide the location of the services API from the client for additional security. The web application uses the same error logging endpoints as the mobile applications for ease of debugging.

### Mobile Applications

Mobile apps can be broadly divided into two categories: data collection / enrollment / linkage (clinician-facing: SA-PHC, COMBIND), and adherence (patient-facing: midot, ebola). In either case, owing to the places deployed and the importance of data integrity, apps assume a store-and-upload model, rather than an attempt-fail-retry model. While failed transmissions need to be handled, the paradigm is more in terms of a hierarchical database that occasionally syncs, and less of an always-online app that only caches things in the event of a connection issue. Apps should not assume that a connection is available, and any operation communicating with the server should check and confirm connection availability before trying to send any data. This system deals with using mobile applications in a connection-less environment. Common issues and patterns:
- Local Storage
   ∘ SQLite is utilized on iOS and Android devices as a durable and structured data store.
   ∘ Pertinent local data storage is also utilized for storing data such as videos temporarily before an upload.
- Login credentials are cached on the device, and as best as possible to allow for a "login" even if the server is unavailable due to lack of connection.
   ∘ Best practice is to, on successful login against services APIs, cache the credentials by concatenating username and password (from the request being sent), and hashing (MD5, SHA1, etc) and stoinge the result in the SQLite database.
   ∘ On following logins, if the server is unreachable, this local table can be utilized to authenticate the user
- Data transmission.
   ∘ Any data to be transmitted - patient enrollment, adherence check-ins, etc, are temporarily stored on a local database table before attempting to send to the server.
   ∘ Timestamps are sent with entered records, and refer to the date/time of device entry, not the time of server receipt.
   ∘ Re-transmission logic is presented in as clear a manner as possible, and react intelligently to specific error codes, to keep things from getting stuck in the cache and repeatedly bouncing them off of the services.
   ∘ The guiding principle is over-transmission of data, as data loss in any amount in unacceptable.
- File uploads
   ∘ Due to recorded video files being large in size, the video data is broken into "chunks" and transmitted to the server incrementally from the mobile device.
   ∘ These videos are part of the adherence check-in events and are sent with timestamps and text data, along with some information on file uploads:
      ▪ Total file size
      ▪ Number of "chunks"
      ▪ Size of each chunk
      ▪ Filename
   ∘ Server returns a file upload ID along with the success message.
   ∘ Each chunk uploads independently, as connection is available, and sends:
      ▪ file upload ID
      ▪ chunk size
      ▪ chunk position in order ("part 1 of 3", eg)
      ▪ binary data
- Encryption
   ∘ All data, whether it's being stored on the device or being sent to services APIs, is encrypted as soon as possible. This uses the AES algorithm, and generates a random Initialization Vector to be prepended to the encrypted payload.
   ∘ Any request that uses a session token is encrypted with the API key for the user attached to that session.

Core functionality (roles and user accounts, common lookup tables) are managed in a central public schema, but each client has their own database, created via scripts as part of the deployment process in order to house their patient information (patient PHI, monitoring check-ins, lab test results, etc).

Audit and error logs are rotated on a monthly basis, out to archive tables (eg, md.audit_logs_04_2015) for performance reasons.

Most "delete" operations are soft-deletes (that is, set the boolean field is_deleted to true, which hides the records in all queries) rather than hard-deletes (actual SQL delete statements). Patients, Users, Checkins, etc must be soft deletes, but other data - sessions, user -> role mappings, can be deleted.

### Multi-stage Compliance progression

In some embodiments, the platform progresses a patient thought multiple stages of compliance. The terms used in the following description are described in Table 1 below:

**Table 1**

| **Term** | | **Definition and Comments** |
|---|---|---|
| Intervention Intensity | | Level of responsiveness by the healthcare worker(s) charged with monitoring the patient. There are three levels: high, medium, low. Each level of intensity has differing requirements for (1) maximum time elapsed between video submission and review, (2) action required in the case of non-compliance; (3) patient performance required to proceed to next stage |
| Compliance Criteria | | All criteria assessed by a User to determine whether a patient-submitted video can be accepted as successful Directly Observed Therapy. Criteria can be broken into two categories: General Process and Critical Criteria. A particular Intervention Intensity or Stage may require that non-compliance relate to a Critical Criteria in order to trigger certain actions. |
| Duration | | The number of days of therapy, or doses, required for a Stage. For example, if a Regimen requires 3x per week medication (MWF), a Stage with a duration of 3 will take 5 calendar days if started on Monday. |
| Stage | | The first few weeks are critical for the healthcare worker to develop strong adherence habits by the patient. This time is broken into four stages. Each stage may have differing Intervention Intensity, duration, Adherence Requirements. |
| Adherence Requirement | | For each regimen, a Stage will have a certain minimum % of successful doses observed in order for the patient to progress to the next Stage. |
| Regimen | | Regimen refers to the number of days per 7-day week a patient is expected to take medication. Regimen also refers to the specific medications that are expected to be observed. Different Regimens may have different Stage durations. |
| Range | | Regimens have a Range which determines up to how many days late an observation can occur. These Ranges are fixed. For a 7-day regimen, the Range = 0. For a 5-day regimen, the range = 2.For a 3-day regimen the Range is 2. For a 1-day regimen the Range is -1 or 3. Example #1: a 3-day regimen requires medication to be taken M-W-F. A patient takes each of these three doses 2 days late: W-F-Sun. The patient has completed 3 doses in 7 days. Example #2: a 5-day regimen. Example #2: A 5-day regimen requires a patient to take medication M-T-W-Th-F. The patient instead takes medication W-Th-F-Sa-Su. The patient has completed 5 doses in a 7-day period. Example #3: a patient has a 1x per week regimen (LTBI) and is required to take their medication every Thursday for 12 weeks total with 4 days minimum in between doses. The medication can be taken as early as Wednesday and as late as Sunday. |
| Target Day | | The Target Day is the specific day a dose is expected. It is possible to achieve 100% adherence while missing the Target Day, provided that doses are taken within the Range. |

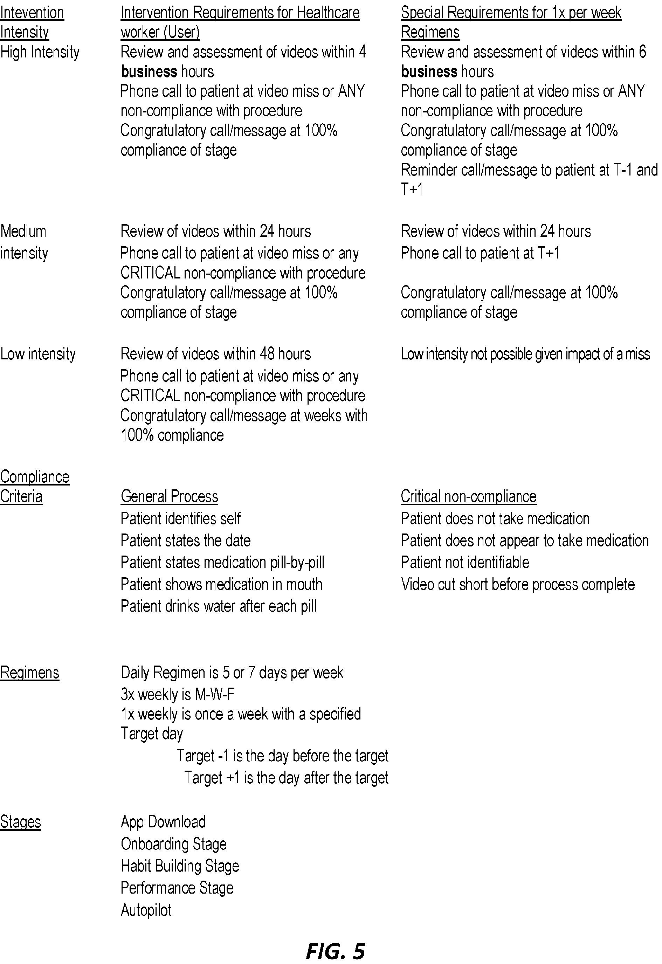

FIG. 5 is a table of the various intervention intensities and compliance criteria according to certain embodiments. As a patient progresses and shows the ability to meet the compliance criteria, the intervention intensity is lowered from high to medium to low. The intervention intensity, as can be seen in FIG. 5, dictates how soon a medical provider needs to review a video for compliance after a patient video is submitted. At high intensity, 4-6 business hours is required, medium intensity requires review within 24 hours and low intensity within 48 hours. These time periods may be varied, with the amount of time being extended for lower intensity interventions. In addition, the intensity level dictates whether a medical provider needs to call the patient after any missed or noncompliant video, only after two errors, etc. Positive feedback to the patient is also provided in the form of a congratulatory call or message upon 100% compliance.

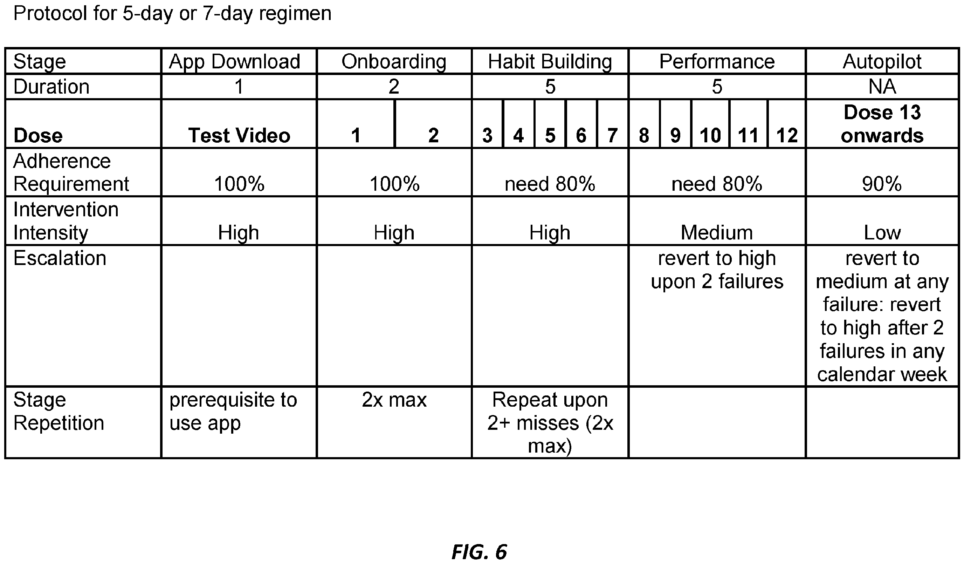

FIG. 6 is a table of the requirements for various stages for a 5-7 day regimen according to certain embodiments. Five stages are shown, (1) app download, (2) onboarding, (3) habit building, (4) performance and (5) autopilot. The adherence requirement starts at 100% for the patient to demonstrate the ability to use the system. The adherence requirement then drops to 80% over a larger number of doses, allowing the patient an occasional slip. In the last stage, the adherence requirement is raised back to 90%, since the intervention intensity will be low. Thus, the patient is required to maintain a high adherence score in order to be trusted to properly take the medication without prompt monitoring by a medical provider.

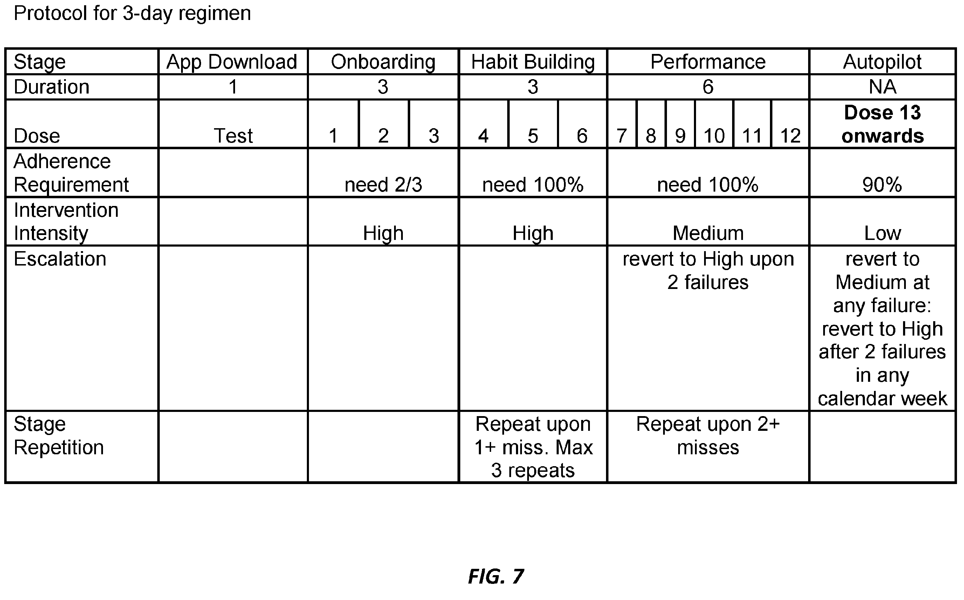

FIG. 7 is a table of the requirements for various stages for a 3 day regimen according to certain embodiments.

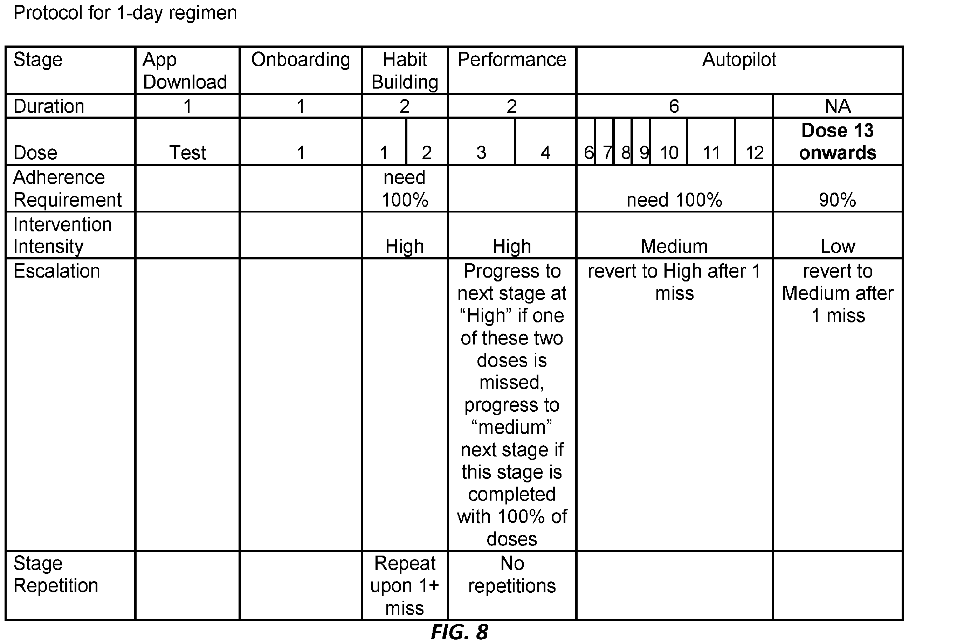

FIG. 8 is a table of the requirements for various stages for a 1 day regimen according to certain embodiments.

### App Download Stage

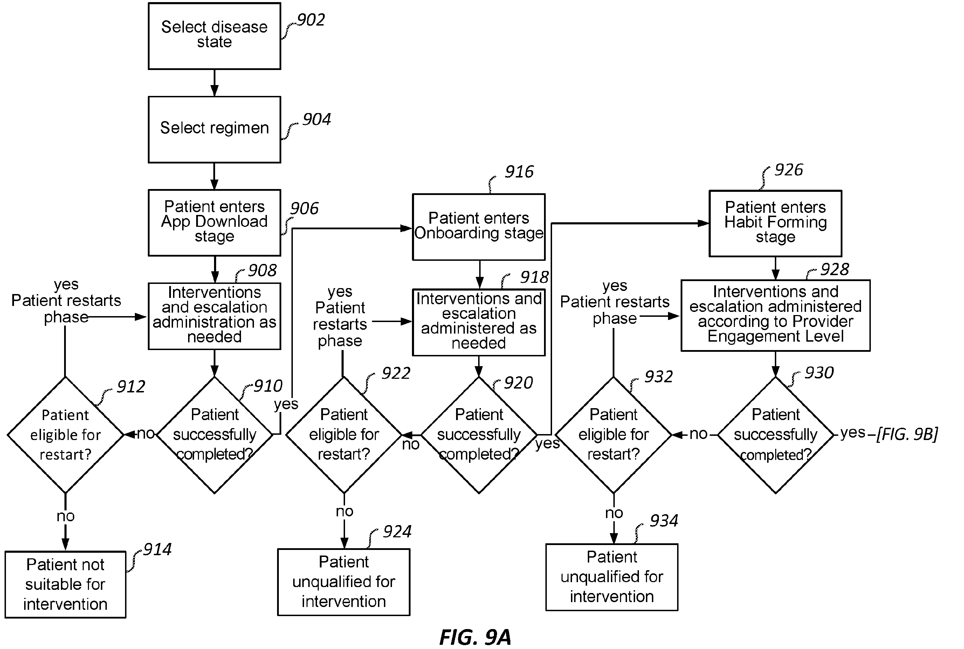

FIGS. 9A-B are a high level flow chart of the stages according to certain embodiments. As shown in FIG. 9A, a disease state (902) and a regimen (904) are selected. The regimen may be the 1, 3 or 5-7 day regimens shown in FIGS. 6-8. This is typically done by the medical provider for a patient. The patient then downloads the app (908) and enters a login and password for access. The patient is prompted to record a test video for upload. It is determined if the patent was successful (910). If the patient fails to properly complete the test upload or otherwise fails to download an operate the app, the patient will be eligible to try again (912). The patient is indicated as unsuitable for using the app (914) after a number of failures, such as 3 failures.

### Onboarding Stage

In the onboarding stage (916), the patient is required to record and upload 1-3 videos depending on the regimen. The patient is typically required to read a script, such as identifying themselves by name and/or birthdate, identifying the medication and dosage, etc. The patient needs to be 100% successful for 1-2 doses, but can be 2/3 successful for 3 doses or more in this stage. Intervention and escalation (918) is administered as needed. It is determined if the patent was successful (920). If the patient fails to properly complete the video upload or otherwise fails to adhere to the protocol (e.g., the video doesn't show the pill going into the patient's mouth, or the patient didn't correctly read the script), the patient will be eligible to try again (922). The patient is indicated as unsuitable for using the app (924) after a number of failures, such as 3 failures.

Through the use of an adequate patient script, the asynchronous review of the video is made possible, without the real-time need for a medical provider to prompt and correct the user. Also, an adequate script enables a simple interaction, without requiring specialized, locking pill containers or other special apparatus. One embodiment of a script is set forth below:
1. State your name and today's date.
2. State the name of the medication.
3. Hold the medication bottle or other packaging up to the camera, showing the label.
4. Open the bottle in view of the camera.
5. Place the medication in your mouth in view of the camera, then drink water or other liquids to show washing it down and swallowing.

### Habit Forming Stage

In the habit forming stage (926), since the patient has already demonstrated the capability to adhere to the regimen, only 80% adherence is required over a larger number of doses, such as 5 doses. Intervention and escalation (928) is administered as needed, per the above-described schedule. It is determined if the patient was successful (930). If the patient fails to properly complete the test upload or otherwise fails to download and operate the app, the patient will be eligible to try again (932). The patient is indicated as unsuitable for using the app (934) after a number of failures, such as 3 failures.

### Performance Stage

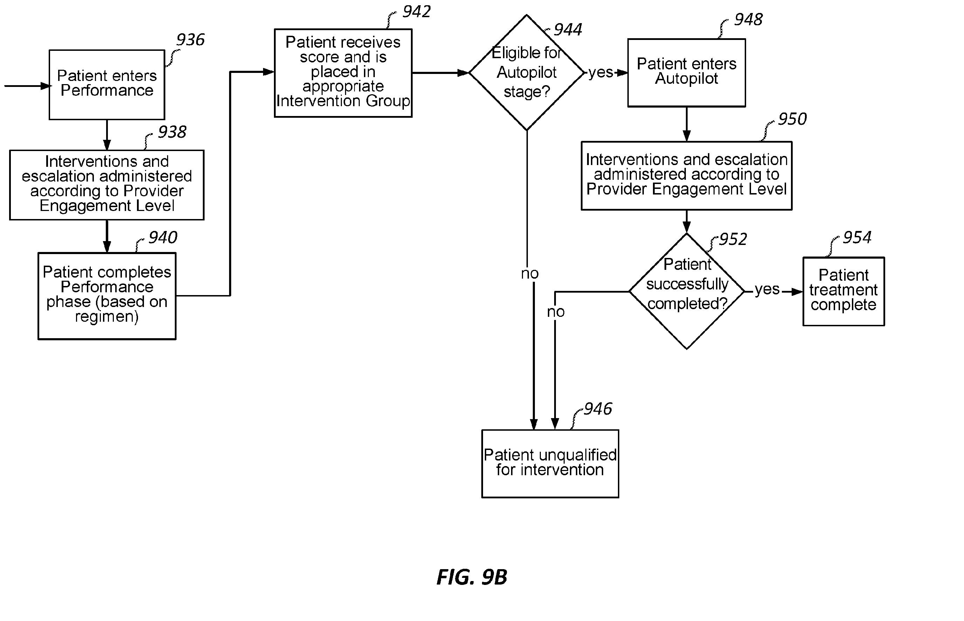

As shown in FIG. 9B, the user then enters the performance stage (936). The performance stage can be considered a continuation of the habit forming stage, but with the intervention intensity lowered to medium, as defined above. The adherence requirement remains at 80%. The patient may then complete the performance stage if the regimen criteria is satisfied (940). Although the steps are not shown in FIG. 9B, the user can remain in the performance stage or be disqualified depending on the number of failures, as for the previous stages.

### Performance Stage

Upon completion of the performance stage, the patient is assigned a score or grade and placed in an appropriate intervention group (942). The grade provides an estimate of future adherence rates based on past performance. A patient with 100% adherence will be placed in a low intensity intervention group, while patients with lower adherence will be placed in medium or high intensity intervention groups. The grade can also be used to determine eligibility (944) instead of allowing the patient multiple tries at passing the performance stage. If the grade is too low, the patient is removed from the program (946). Otherwise, the patient enters the autopilot stage (948) at the assigned intervention level. Interventions and escalations are administered according to the medical provider's engagement level (950). If the patient successfully completes the autopilot stage (952), the patient treatment is complete (954). Otherwise, the patient is unqualified for intervention (946) and is removed from the program.

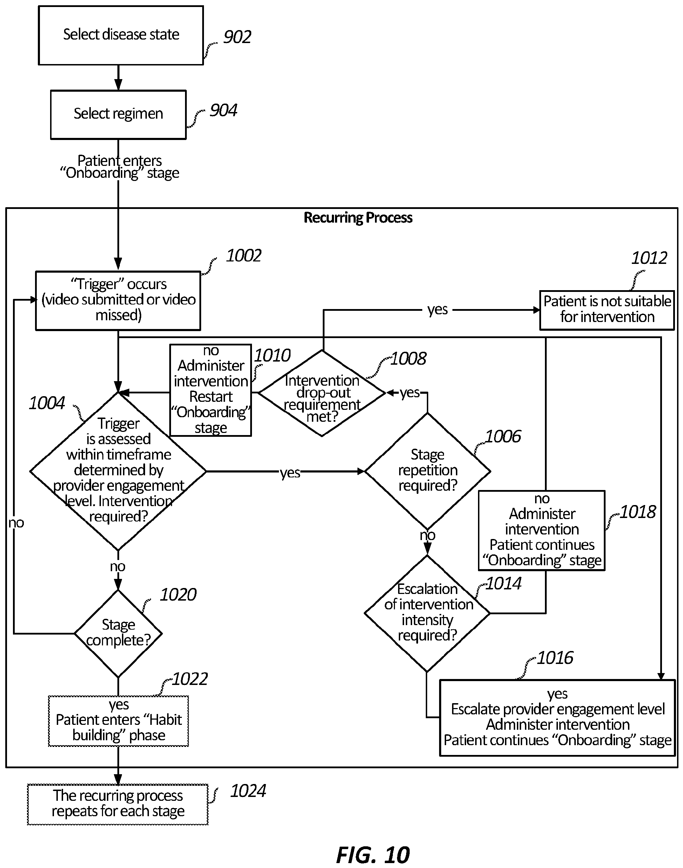

FIG. 10 is a more detailed flow chart of a generic stage according to certain embodiments. FIG. 10 illustrates the recurring process for the example between app download and onboarding, but is applicable to the process, which recurs, between other stages. A trigger occurs (1002) in the stage, such as a video being submitted, or a video being missed and the time period has expired for submitting the video. Upon review by a medical provider, it is determined whether provider intervention or engagement is required (1004). If engagement is required, it is determined whether stage repetition is required (1006). If repetition is required, it is determined if the patient has failed the maximum allowed number of times (1008). If not, the stage is restarted (1010), with the sytem automatically restarting or reseting the onboarding or other stage. If the maximum number of failures has been reached, the patient is not suitable for intervention (1012).

If no stage repetition is required (1006), it is determined if escalation of intervention intensity is required (1014). If no intervention is administered and the patient continues the stage (1018). If escalation is required, the provider engagement level is escalated and intervention is administered accordingly (1016). The patient then continues the stage.

### Medical Provider Interface

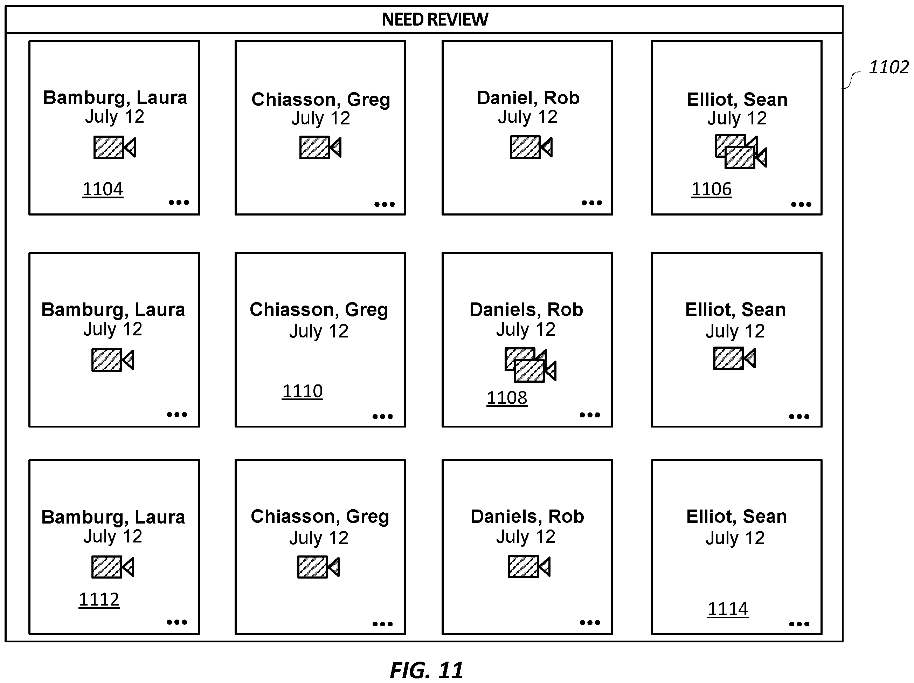

FIG. 11 is a diagram of a medical provider triaged review interface 1102 according to certain embodiments. This diagram illustrates some concepts, and a variety of different interfaces could be used, with different manners of arranging and displaying the data. The interface shows uploaded patient videos ready for review, with a first video 1104 being the one triaged to be the most important to review first. Where a patient has multiple videos for review, this is indicated by multiple video camera icons for patients videos 1106 and 1108. A number of patients are overdue submitting videos, and thus no video icon is shown for patients 1110, 1112 and 1114. In addition, the border of these boxes may be highlighted in red, or another form of emphasis can be provided. Videos for different patients are thus queued up for review based on the patient adherence intensity level. The queue is updated in real time as missing or late patient videos are received, or as partial video uploads are completed. The order of the presented videos are thus continuously updated, invisibly to the medical provider, so that when the medical provider logs on and starts reviewing the videos, they are in the real-time, appropriately triaged order.

The PROVIDER ENGAGEMENT INTENSITY (PEI) varies automatically based on the patient's performance. The PEI has three categories: high, medium, low. Actions and interventions are programmed for each category. Sample pre-determined actions:
∘ review patient video within X hours
∘ call patient in certain circumstances
   □ failure to submit a video
   □ submitting a non-compliant video
   □ good performance

- Providers are prompted by the system to interact with the patient at certain times On the provider interface, videos are sorted based on the PEI level. Because the PEI level determines the maximum delay between video submission and video review, the videos can be sorted in order of time left for the provider to review, which will usually mirror the PEI.

In one embodiment, additional information is provided on a medical provider interface, with the ability to drill down for more information. For example, an interface display can set forth a list of the patient videos needing review. The list includes patients who have not uploaded a video in the designated time period. The list can be dynamically modified as patient videos come in later, with a high priority patient being re-categorized as lower priority with a late arriving video. The interface can indicate the time the video was recorded, the time of submission, any side effects indicated by the patient, the numerical progress through the dosage regimen, the current adherence percentage and raw numbers (e.g., 2/5). The interface will also indicate when videos have been reviewed.

In one embodiment, based on the medical provider's inputs, the medical provider is prompted to undertake certain interactions depending upon the stage and adherence as described above. For example, if a video is marked as non-compliant, the medical provider may be prompted to call the patient depending on the patient's adherence record, stage, and the type of non-compliance. For a major non-compliance, such as the wrong medication, or the wrong patient name, the medical provider may be prompted to call the patient immediately. For a minor non-compliance, such as a minor error in reading the script, the medical provider may not be prompted to call, or may be prompted to call within the next week as opposed to immediately.

### Computer Systems for Client Server System

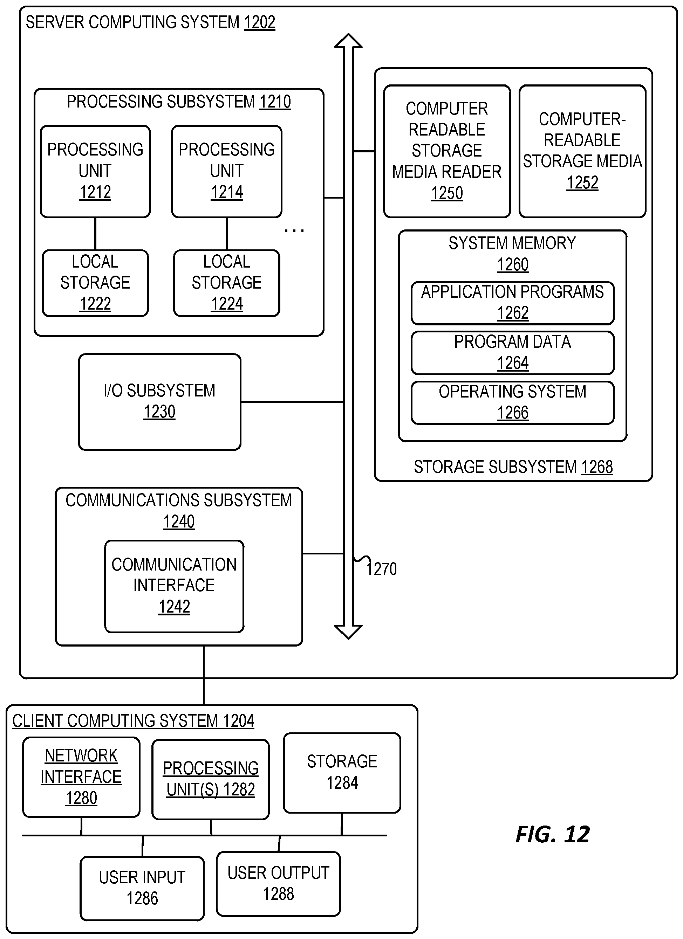

Various operations described herein may be implemented on computer systems. FIG. 12 shows a simplified block diagram of a representative computing system 1202 and client computing system 1204 usable to implement certain embodiments of the present invention. In various embodiments, computing system 1202 or similar systems may implement the remote server, or any other computing system described herein or portions thereof. Client computing system 1204 or similar systems may implement user devices such as a smartphone or other user device.

Computing system 1202 may be one of various types, including processor and memory, a handheld portable device (e.g., an iPhone^{®} cellular phone, an iPad^{®} computing tablet, a PDA), a wearable device (e.g., a Google Glass^{®} head mounted display), a personal computer, a workstation, a mainframe, a kiosk, a server rack, or any other data processing system.

Computing system 1202 may include processing subsystem 1210. Processing subsystem 1210 may communicate with a number of peripheral systems via bus subsystem 1270. These peripheral systems may include I/O subsystem 1230, storage subsystem 1268, and communications subsystem 1240.

Bus subsystem 1270 provides a mechanism for letting the various components and subsystems of server computing system 1204 communicate with each other as intended. Although bus subsystem 1270 is shown schematically as a single bus, alternative embodiments of the bus subsystem may utilize multiple buses. Bus subsystem 1270 may form a local area network that supports communication in processing subsystem 1210 and other components of server computing system 1202. Bus subsystem 1270 may be implemented using various technologies including server racks, hubs, routers, etc. Bus subsystem 1270 may be any of several types of bus structures including a memory bus or memory controller, a peripheral bus, and a local bus using any of a variety of bus architectures. For example, such architectures may include an Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus, which may be implemented as a Mezzanine bus manufactured to the IEEE P1386.1 standard, and the like.

I/O subsystem 1230 may include devices and mechanisms for inputting information to computing system 1202 and/or for outputting information from or via computing system 1202. In general, use of the term "input device" is intended to include all possible types of devices and mechanisms for inputting information to computing system 1202. User interface input devices may include, for example, a keyboard, pointing devices such as a mouse or trackball, a touchpad or touch screen incorporated into a display, a scroll wheel, a click wheel, a dial, a button, a switch, a keypad, audio input devices with voice command recognition systems, microphones, and other types of input devices. User interface input devices may also include motion sensing and/or gesture recognition devices such as the Microsoft Kinect^{®} motion sensor that enables users to control and interact with an input device, the Microsoft Xbox^{®} 360 game controller, devices that provide an interface for receiving input using gestures and spoken commands. User interface input devices may also include eye gesture recognition devices such as the Google Glass^{®} blink detector that detects eye activity (e.g., "blinking" while taking pictures and/or making a menu selection) from users and transforms the eye gestures as input into an input device (e.g., Google Glass^{®}). Additionally, user interface input devices may include voice recognition sensing devices that enable users to interact with voice recognition systems (e.g., Siri^{®} navigator), through voice commands.

Other examples of user interface input devices include, without limitation, three dimensional (3D) mice, joysticks or pointing sticks, gamepads and graphic tablets, and audio/visual devices such as speakers, digital cameras, digital camcorders, portable media players, webcams, image scanners, fingerprint scanners, barcode reader 3D scanners, 3D printers, laser rangefinders, and eye gaze tracking devices. Additionally, user interface input devices may include, for example, medical imaging input devices such as computed tomography, magnetic resonance imaging, position emission tomography, medical ultrasonography devices. User interface input devices may also include, for example, audio input devices such as MIDI keyboards, digital musical instruments and the like.

User interface output devices may include a display subsystem, indicator lights, or non-visual displays such as audio output devices, etc. The display subsystem may be a cathode ray tube (CRT), a flat-panel device, such as that using a liquid crystal display (LCD) or plasma display, a projection device, a touch screen, and the like. In general, use of the term "output device" is intended to include all possible types of devices and mechanisms for outputting information from computing system 1202 to a user or other computer. For example, user interface output devices may include, without limitation, a variety of display devices that visually convey text, graphics and audio/video information such as monitors, printers, speakers, headphones, automotive navigation systems, plotters, voice output devices, and modems.

Processing subsystem 1210 controls the operation of computing system 1202 and may comprise one or more processing units 1212, 1214, etc. A processing unit may include one or more processors, including single core processor or multicore processors, one or more cores of processors, or combinations thereof. In some embodiments, processing subsystem 1210 may include one or more special purpose co-processors such as graphics processors, digital signal processors (DSPs), or the like. In some embodiments, some or all of the processing units of processing subsystem 1210 may be implemented using customized circuits, such as application specific integrated circuits (ASICs), or field programmable gate arrays (FPGAs). In some embodiments, such integrated circuits execute instructions that are stored on the circuit itself. In other embodiments, processing unit(s) may execute instructions stored in local storage, e.g., local storage 1222, 1224. Any type of processors in any combination may be included in processing unit(s) 1212, 1214.

In some embodiments, processing subsystem 1210 may be implemented in a modular design that incorporates any number of modules (e.g., blades in a blade server implementation). Each module may include processing unit(s) and local storage. For example, processing subsystem 1210 may include processing unit 1212 and corresponding local storage 1222, and processing unit 1214 and corresponding local storage 1224.

Local storage 1222, 1224 may include volatile storage media (e.g., conventional DRAM, SRAM, SDRAM, or the like) and/or non-volatile storage media (e.g., magnetic or optical disk, flash memory, or the like). Storage media incorporated in local storage 1222, 1224 may be fixed, removable or upgradeable as desired. Local storage 1222, 1224 may be physically or logically divided into various subunits such as a system memory, a ROM, and a permanent storage device. The system memory may be a read-and-write memory device or a volatile read-and-write memory, such as dynamic random access memory. The system memory may store some or all of the instructions and data that processing unit(s) 1212, 1214 need at runtime. The ROM may store static data and instructions that are needed by processing unit(s) 1212, 1214. The permanent storage device may be a non-volatile read-and-write memory device that may store instructions and data even when a module including one or more processing units 1212, 1214 and local storage 1222, 1224 is powered down. The term "storage medium" as used herein includes any medium in which data may be stored indefinitely (subject to overwriting, electrical disturbance, power loss, or the like) and does not include carrier waves and transitory electronic signals propagating wirelessly or over wired connections.

In some embodiments, local storage 1222, 1224 may store one or more software programs to be executed by processing unit(s) 1212, 1214, such as an operating system and/or programs implementing various server functions such as functions of UPP system 102, or any other server(s) associated with UPP system 102. "Software" refers generally to sequences of instructions that, when executed by processing unit(s) 1212, 1214 cause computing system 1202 (or portions thereof) to perform various operations, thus defining one or more specific machine implementations that execute and perform the operations of the software programs. The instructions may be stored as firmware residing in read-only memory and/or program code stored in non-volatile storage media that may be read into volatile working memory for execution by processing unit(s) 1212, 1214. In some embodiments the instructions may be stored by storage subsystem 1268 (e.g., computer readable storage media). In various embodiments, the processing units may execute a variety of programs or code instructions and may maintain multiple concurrently executing programs or processes. At any given time, some or all of the program code to be executed may be resident in local storage 1222, 1224 and/or in storage subsystem including potentially on one or more storage devices. Software may be implemented as a single program or a collection of separate programs or program modules that interact as desired. From local storage 1222, 1224 (or non-local storage described below), processing unit(s) 1212, 1214 may retrieve program instructions to execute and data to process in order to execute various operations described above.

Storage subsystem 1268 provides a repository or data store for storing information that is used by computing system 1202. Storage subsystem 1268 provides a tangible non-transitory computer-readable storage medium for storing the basic programming and data constructs that provide the functionality of some embodiments. Software (programs, code modules, instructions) that when executed by processing subsystem 1210 provide the functionality described above may be stored in storage subsystem 1268. The software may be executed by one or more processing units of processing subsystem 1210. Storage subsystem 1268 may also provide a repository for storing data used in accordance with the present invention.

Storage subsystem 1268 may include one or more non-transitory memory devices, including volatile and non-volatile memory devices. As shown in FIG. 12, storage subsystem 1268 includes a system memory 1260 and a computer-readable storage media 1252. System memory 1260 may include a number of memories including a volatile main RAM for storage of instructions and data during program execution and a non-volatile ROM or flash memory in which fixed instructions are stored. In some implementations, a basic input/output system (BIOS), containing the basic routines that help to transfer information between elements within computing system 1202, such as during start-up, may typically be stored in the ROM. The RAM typically contains data and/or program modules that are presently being operated and executed by processing subsystem 1210. In some implementations, system memory 1260 may include multiple different types of memory, such as static random access memory (SRAM) or dynamic random access memory (DRAM). Storage subsystem 1268 may be based on magnetic, optical, semiconductor, or other data storage media. Direct attached storage, storage area networks, network-attached storage, and the like may be used. Any data stores or other collections of data described herein as being produced, consumed, or maintained by a service or server may be stored in storage subsystem 1268.

By way of example, and not limitation, as depicted in FIG. 12, system memory 1260 may store application programs 1262, which may include client applications, Web browsers, mid-tier applications, relational database management systems (RDBMS), etc., program data 1264, and one or more operating systems 1266. By way of example, an example operating systems may include various versions of Microsoft Windows^{®}, Apple Macintosh^{®}, and/or Linux operating systems, a variety of commercially-available UNIX^{®} or UNIX-like operating systems (including without limitation the variety of GNU/Linux operating systems, the Google Chrome^{®} OS, and the like) and/or mobile operating systems such as iOS, Windows^{®} Phone, Android^{®} OS, BlackBerry^{®} 10 OS, and Palm^{®} OS operating systems.

Computer-readable storage media 1252 may store programming and data constructs that provide the functionality of some embodiments. Software (programs, code modules, instructions) that when executed by processing subsystem 1210 a processor provide the functionality described above may be stored in storage subsystem 1268. By way of example, computer-readable storage media 1252 may include non-volatile memory such as a hard disk drive, a magnetic disk drive, an optical disk drive such as a CD ROM, DVD, a Blu-Ray^{®} disk, or other optical media. Computer-readable storage media 1252 may include, but is not limited to, Zip^{®} drives, flash memory cards, universal serial bus (USB) flash drives, secure digital (SD) cards, DVD disks, digital video tape, and the like. Computer-readable storage media 1252 may also include, solid-state drives (SSD) based on non-volatile memory such as flash-memory based SSDs, enterprise flash drives, solid state ROM, and the like, SSDs based on volatile memory such as solid state RAM, dynamic RAM, static RAM, DRAM-based SSDs, magnetoresistive RAM (MRAM) SSDs, and hybrid SSDs that use a combination of DRAM and flash memory based SSDs. Computer-readable media 1252 may provide storage of computer-readable instructions, data structures, program modules, and other data for computing system 1202.

In certain embodiments, storage subsystem 1268 may also include a computer-readable storage media reader 1250 that may further be connected to computer-readable storage media 1252. Together and, optionally, in combination with system memory 1260, computer-readable storage media 1252 may comprehensively represent remote, local, fixed, and/or removable storage devices plus storage media for storing computer-readable information.

In certain embodiments, computing system 1202 may provide support for executing one or more virtual machines. Computing system 1202 may execute a program such as a hypervisor for facilitating the configuring and managing of the virtual machines. Each virtual machine may be allocated memory, compute (e.g., processors, cores), I/O, and networking resources. Each virtual machine typically runs its own operating system, which may be the same as or different from the operating systems executed by other virtual machines executed by computing system 1202. Accordingly, multiple operating systems may potentially be run concurrently by computing system 1202. Each virtual machine generally runs independently of the other virtual machines.

Communication subsystem 1240 provides an interface to other computer systems and networks. Communication subsystem 1240 serves as an interface for receiving data from and transmitting data to other systems from computing system 1202. For example, communication subsystem 1240 may enable computing system 1202 to establish a communication channel to one or more client computing devices via the Internet for receiving and sending information from and to the client computing devices.

Communication subsystem 1240 may support both wired and/or wireless communication protocols. For example, in certain embodiments, communication subsystem 1240 may include radio frequency (RF) transceiver components for accessing wireless voice and/or data networks (e.g., using cellular telephone technology, advanced data network technology, such as 3G, 4G or EDGE (enhanced data rates for global evolution), WiFi (IEEE 802.11 family standards, or other mobile communication technologies, or any combination thereof), global positioning system (GPS) receiver components, and/or other components. In some embodiments communication subsystem 1240 may provide wired network connectivity (e.g., Ethernet) in addition to or instead of a wireless interface.

Communication subsystem 1240 may receive and transmit data in various forms. For example, in some embodiments, communication subsystem 1240 may receive input communication in the form of structured and/or unstructured data feeds, event streams, event updates, and the like. For example, communication subsystem 1240 may be configured to receive (or send) data feeds in real-time from users of social media networks and/or other communication services such as Twitter^{®} feeds, Facebook^{®} updates, web feeds such as Rich Site Summary (RSS) feeds, and/or real-time updates from one or more third party information sources.

In certain embodiments, communication subsystem 1240 may be configured to receive data in the form of continuous data streams, which may include event streams of real-time events and/or event updates, that may be continuous or unbounded in nature with no explicit end. Examples of applications that generate continuous data may include, for example, sensor data applications, financial tickers, network performance measuring tools (e.g. network monitoring and traffic management applications), clickstream analysis tools, automobile traffic monitoring, and the like.

Communication subsystem 1240 may also be configured to output the structured and/or unstructured data feeds, event streams, event updates, and the like to one or more databases that may be in communication with one or more streaming data source computers coupled to computing system 1202.

Communication subsystem 1240 may provide a communication interface 1242, e.g., a WAN interface, which may provide data communication capability between the local area network (bus subsystem 1270) and a larger network, such as the Internet. Conventional or other communications technologies may be used, including wired (e.g., Ethernet, IEEE 802.3 standards) and/or wireless technologies (e.g., Wi-Fi, IEEE 802.11 standards).

Computing system 1202 may operate in response to requests received via communication interface 1242. Further, in some embodiments, communication interface 1242 may connect computing systems 1202 to each other, providing scalable systems capable of managing high volumes of activity. Conventional or other techniques for managing server systems and server farms (collections of server systems that cooperate) may be used, including dynamic resource allocation and reallocation.

Computing system 1202 may interact with various user-owned or user-operated devices via a wide-area network such as the Internet. An example of a user-operated device is shown in FIG. 12 as client computing system 1202. Client computing system 1204 may be implemented, for example, as a consumer device such as a smart phone, other mobile phone, tablet computer, wearable computing device (e.g., smart watch, eyeglasses), desktop computer, laptop computer, and so on.

For example, client computing system 1204 may communicate with computing system 1202 via communication interface 1242. Client computing system 1204 may include conventional computer components such as processing unit(s) 1282, storage device 1284, network interface 1280, user input device 1286, and user output device 1288. Client computing system 1204 may be a computing device implemented in a variety of form factors, such as a desktop computer, laptop computer, tablet computer, smart phone, other mobile computing device, wearable computing device, or the like.

Processing unit(s) 1282 and storage device 1284 may be similar to processing unit(s) 1212, 1214 and local storage 1222, 1224 described above. Suitable devices may be selected based on the demands to be placed on client computing system 1204; for example, client computing system 1204 may be implemented as a "thin" client with limited processing capability or as a high-powered computing device. Client computing system 1204 may be provisioned with program code executable by processing unit(s) 1282 to enable various interactions with computing system 1202 of a message management service such as accessing messages, performing actions on messages, and other interactions described above. Some client computing systems 1204 may also interact with a messaging service independently of the message management service.

Network interface 1280 may provide a connection to a wide area network (e.g., the Internet) to which communication interface 1240 of computing system 1202 is also connected. In various embodiments, network interface 1280 may include a wired interface (e.g., Ethernet) and/or a wireless interface implementing various RF data communication standards such as Wi-Fi, Bluetooth^{®}, or cellular data network standards (e.g., 3G, 4G, LTE, etc.).

User input device 1286 may include any device (or devices) via which a user may provide signals to client computing system 1204; client computing system 1204 may interpret the signals as indicative of particular user requests or information. In various embodiments, user input device 1286 may include any or all of a keyboard, touch pad, touch screen, mouse or other pointing device, scroll wheel, click wheel, dial, button, switch, keypad, microphone, and so on.

User output device 1288 may include any device via which client computing system 1204 may provide information to a user. For example, user output device 1288 may include a display to display images generated by or delivered to client computing system 1204. The display may incorporate various image generation technologies, e.g., a liquid crystal display (LCD), light-emitting diode (LED) including organic light-emitting diodes (OLED), projection system, cathode ray tube (CRT), or the like, together with supporting electronics (e.g., digital-to-analog or analog-to-digital converters, signal processors, or the like). Some embodiments may include a device such as a touchscreen that function as both input and output device. In some embodiments, other user output devices 1288 may be provided in addition to or instead of a display. Examples include indicator lights, speakers, tactile "display" devices, printers, and so on.

Some embodiments include electronic components, such as microprocessors, storage and memory that store computer program instructions in a computer readable storage medium. Many of the features described in this specification may be implemented as processes that are specified as a set of program instructions encoded on a computer readable storage medium. When these program instructions are executed by one or more processing units, they cause the processing unit(s) to perform various operation indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 1212, 1214 and 1282 may provide various functionality for computing system 1202 and client computing system 1204, including any of the functionality described herein as being performed by a server or client, or other functionality associated with message management services.

It will be appreciated that computing system 1202 and client computing system 1204 are illustrative and that variations and modifications are possible. Computer systems used in connection with embodiments of the present invention may have other capabilities not specifically described here. Further, while computing system 1202 and client computing system 1204 are described with reference to particular blocks, it is to be understood that these blocks are defined for convenience of description and are not intended to imply a particular physical arrangement of component parts. For instance, different blocks may be but need not be located in the same facility, in the same server rack, or on the same motherboard. Further, the blocks need not correspond to physically distinct components. Blocks may be configured to perform various operations, e.g., by programming a processor or providing appropriate control circuitry, and various blocks might or might not be reconfigurable depending on how the initial configuration is obtained. Embodiments of the present invention may be realized in a variety of apparatus including electronic devices implemented using any combination of circuitry and software.

While the invention has been described with respect to specific embodiments, one skilled in the art will recognize that numerous modifications are possible. Embodiments of the invention may be realized using a variety of computer systems and communication technologies including but not limited to specific examples described herein.

Embodiments of the present invention may be realized using any combination of dedicated components and/or programmable processors and/or other programmable devices. The various processes described herein may be implemented on the same processor or different processors in any combination. Where components are described as being configured to perform certain operations, such configuration may be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present invention may be encoded and stored on various computer readable storage media; suitable media include magnetic disk or tape, optical storage media such as compact disk (CD) or DVD (digital versatile disk), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the invention has been described with respect to specific embodiments, it will be appreciated that the invention is intended to cover all modifications and equivalents within the scope of the following claims.

While certain exemplary embodiments have been described in detail and shown in the accompanying drawings, it is to be understood that such embodiments are merely illustrative of and not intended to be restrictive of the broad invention, and that this invention is not to be limited to the specific arrangements and constructions shown and described, since various other modifications may occur to those with ordinary skill in the art.

Other variations are within the spirit of the present disclosure. Thus, while the disclosed techniques are susceptible to various modifications and alternative constructions, certain illustrated embodiments thereof are shown in the drawings and have been described above in detail. It should be understood, however, that there is no intention to limit the disclosure to the specific form or forms disclosed, but on the contrary, the intention is to cover all modifications, alternative constructions and equivalents falling within the spirit and scope of the disclosure, as defined in the appended claims.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if there is something intervening. The phrase "based on" should be understood to be open-ended, and not limiting in any way, and is intended to be interpreted or otherwise read as "based at least in part on," where appropriate. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate embodiments of the disclosure and does not pose a limitation on the scope of the disclosure unless otherwise claimed. No language in the specification should be construed as indicating any non-claimed element as essential to the practice of the disclosure.

## Claims

1. A computer-implemented method comprising:
receiving, by a processor, dosage data corresponding to a medication dosage regimen for a medication prescribed to a patient;
receiving, by the processor from a patient's remote mobile device, one or more video data files recorded on the patient's mobile device, including video of the patient administering the medication at intervals defined by the medication dosage regimen;
receiving a request from a medical provider to access the one or more video data files;
providing the medical provider access to the one or more video data files;
receiving approval data corresponding to the medical provider's acceptance or rejection of the one or more video data files as proof of the patient taking a correct dosage of the medication as defined in the medication dosage regimen;
calculating a medication regimen adherence value based on the medication dosage regimen and the approval data;
wherein the medical provider is able to review multiple video data files at a time,
wherein the patient mobile device has an application loaded for secure access with a password and encryption capability wherein, before allowing the patient viewing patient information, patient's permissions are checked to determine whether the patient is allowed to access an object in question, and wherein the patient cannot access the recorded video data files on the patient mobile device using a standard system media library while they are stored on the patient mobile device; and
wherein the recorded video data files are encrypted to prevent access to unauthorized viewers using directory management software.

2. The method of claim 1 wherein the calculating a medication regimen adherence value is performed by a system program separate from a medical provider program on a medical provider device and/or wherein the video data files each include a time and date stamp of a time and date of recording.

3. The method of claim 1 further comprising:
modifying a medical provider intervention intensity based on the medication regimen adherence value, wherein the intervention intensity comprises an amount of time after receipt of the patient video at the secure patient database for the medical provider to review the patient video, in particular, wherein modifying a medical provider intervention intensity based on the medication regimen adherence value comprises advancing the patient to a subsequent monitoring stage, wherein a final monitoring stage requires a lesser intensity of intervention.

4. The method of claim 3, wherein a first stage comprises:
indicating successful downloading of the application,
verifying a patient password,
capturing a patient test video,
encrypting the patient test video,
establishing a secure connection over the network to the secure patient database, and
uploading an encrypted patient test video to the secure patient database, or
wherein a first stage comprises
receiving a medical provider input verifying an adherence by the patient to the medication dosage regimen for a predetermined number of dosages, and
indicating an advancement of the patient to a subsequent stage.

5. The method of claim 3, wherein the operations include advancing a patient through multiple stages requiring high, medium and low values of medical provider intervention intensity.

6. The method of claim 1 further comprising:
encrypting the video data file,
establishing a secure connection over a network to a secure patient database,
uploading an encrypted video data file to the secure patient database.

7. A system for securing medication adherence remotely over a network in communication with a patient computing device and a medical provider computing device, comprising:
at least one secure patient database storing
encrypted patient profiles including medication descriptions, medication dosages, and dosage regimens,
encrypted patient videos of patients taking medications;
a patient program non-transitory computer-readable storage medium containing instructions configured to cause a processor in the patient computing device to perform operations including
verifying a patient password,
recording one or more video data files including a patient video showing the patient taking a medication,
encrypting the patient video,
establishing a secure connection over the network to the secure patient database, uploading an encrypted patient video to the secure patient database;
a medical provider program non-transitory computer-readable storage medium containing instructions configured to cause a processor in the medical provider computing device to perform operations including
verifying a medical provider password,
establishing a secure connection over the network to the secure patient database, displaying information relating to multiple patients in a triaged order,
accessing an encrypted patient video in the secure patient database in response to a selection by a medical provider,
decrypting the encrypted patient video,
receiving approval data corresponding to the medical provider's acceptance or rejection of the one or more video data files as proof of the patient taking a correct dosage of the medication as defined in a medication dosage regimen;
a system program non-transitory computer-readable storage medium containing instructions configured to cause a processor to perform operations including
calculating a medication regimen adherence value based on the medication dosage regimen and the approval data; and
modifying a medical provider intervention intensity based on the medication regimen adherence value,
wherein the patient computing device has an application loaded for secure access with a password and encryption capability, wherein, before allowing the patient viewing patient information, the patient's permissions are checked to determine whether the patient is allowed to access an object in question, and wherein the patient cannot access the recorded video data files on the patient computing device using a standard system media library while they are stored on the patient computing device; and
wherein the recorded video data files are encrypted to prevent access to unauthorized viewers using directory management software.

8. The system of claim 7 wherein the system program non-transitory computer-readable storage medium is part of the medical provider program non-transitory computer-readable storage medium.

9. The system of claim 7 wherein the patient videos each include a time and date stamp of a time and date of recording, and/or wherein the intervention intensity comprises an amount of time after receipt of the patient video at the secure patient database for the medical provider to review the patient video.

10. The system of claim 7 wherein modifying a medical provider intervention intensity based on the medication regimen adherence value comprises advancing the patient to a subsequent monitoring stage, wherein a final monitoring stage requires a lesser intensity of intervention.

11. The system of claim 10 wherein a first stage comprises instructions in the patient program non-transitory computer-readable storage medium configured to cause the processor in the patient computing device to perform operations including
indicating successful downloading of the patient program,
verifying a patient password,
capturing a patient test video,
encrypting the patient test video,
establishing a secure connection over the network to the secure patient database,
uploading an encrypted patient test video to the secure patient database.

12. The system of claim 10 wherein a first stage comprises instructions in the medical provider program non-transitory computer-readable storage medium configured to cause the processor in the medical provider computing device to perform operations including
receiving a medical provider input verifying an adherence by the patient to the medication dosage regimen for a predetermined number of dosages,
indicating an advancement of the patient to a subsequent stage.

13. The system of claim 10 wherein the operations include advancing a patient through multiple stages requiring high, medium and low values of medical provider intervention intensity.

14. The system of claim 7 wherein the network comprises the internet and the secure connection over the network comprises an SSL tunnel.

15. The system according to claim 11,
wherein the patient video includes a time stamp of a time of recording,
wherein establishing the respective secure connection comprises establishing a secure SSL tunnel connection over the network to the secure patient database,
wherein the intervention intensity comprises an amount of time after receipt of the patient video at the secure patient database for the medical provider to review the patient video, wherein the modifying comprises advancing the patient to a subsequent monitoring stage, wherein a final monitoring stage modifies the intensity of intervention.

## Patentansprüche

1. Computerimplementiertes Verfahren, das umfasst:
Empfangen von Dosierungs-Daten, die einem Medikamenten-Verabreichungsplan für ein einem Patienten verschriebenes Medikament entsprechen, durch einen Prozessor;
Empfangen einer oder mehrerer Videodaten-Datei/en, die auf einem mobilen Gerät eines Patienten aufgezeichnet ist/sind und Videoaufnahmen des Patienten einschließt/einschließen, der das Medikament einnimmt, durch den Prozessor von dem entfernten mobilen Gerät des Patienten in Intervallen, die durch den Medikamenten-Verabreichungsplan bestimmt werden;
Empfangen einer Anforderung zum Zugreifen auf die eine oder die mehreren Videodaten-Datei/en von einem medizinischen Dienstleister;
Gewähren von Zugriff auf die eine oder die mehrere/n Videodaten-Datei/en für den medizinischen Dienstleister;
Empfangen von Zustimmungs-Daten, die Annahme oder Ablehnung der einen oder mehreren Videodaten-Datei/en als Beleg dafür, dass der Patient eine Dosierung des Medikaments einnimmt, wie sie in dem Medikamenten-Verabreichungsplan bestimmt ist, durch den medizinischen Dienstleister entsprechen;
Berechnen eines Wertes für Einhaltung des Medikationsplans auf Basis des Medikamenten-Verabreichungsplans und der Zustimmungs-Daten;
wobei der medizinische Dienstleister in der Lage ist, mehrere Videodateien gleichzeitig einzusehen,
wobei auf das mobile Gerät des Patienten eine Anwendung für sicheren Zugang mit einem Passwort und Verschlüsselungsmöglichkeit geladen wird, und bevor dem Patienten erlaubt wird, Patienten-Informationen einzusehen, Berechtigungen des Patienten überprüft werden, um festzustellen, ob dem Patienten gestattet wird, auf ein betreffendes Objekt zuzugreifen, und wobei der Patient nicht auf die aufgezeichneten Videodaten-Dateien auf dem mobilen Gerät des Patienten unter Verwendung einer standardgemäßen System-Medienbibliothek zugreifen kann, während sie auf dem mobilen Gerät des Patienten gespeichert sind; und
wobei die aufgezeichneten Videodaten-Dateien verschlüsselt werden, um Zugriff unbefugter Betrachter unter Verwendung von Verzeichnisverwaltungs-Software zu verhindern.

2. Verfahren nach Anspruch 1, wobei das Berechnen eines Wertes für Einhaltung des Medikationsplans von einem Systemprogramm separat von einem Programm des medizinischen Dienstleisters auf einem Gerät des medizinischen Dienstleisters durchgeführt wird, und/oder wobei die Videodaten-Dateien jeweils einen Zeit-und-Datums-Stempel einer Zeit und eines Datums der Aufnahme enthalten.

3. Verfahren nach Anspruch 1, das des Weiteren umfasst:
Modifizieren einer Interventions-Intensität des medizinischen Dienstleisters auf Basis des Wertes für Einhaltung des Medikationsplans, wobei die Interventions-Intensität eine Zeitspanne nach Empfang des Patienten-Videos an der sicheren Patienten-Datenbank zum Einsehen des Patienten-Videos für den medizinischen Dienstleister umfasst, und insbesondere Modifizieren einer Interventions-Intensität des medizinischen Dienstleisters auf Basis des Wertes für Einhaltung des Medikationsplans umfasst, dass der Patient zu einer nachfolgenden Überwachungsstufe weitergeführt wird, wobei eine abschließende Überwachungsstufe eine geringere Interventions-Intensität erfordert.

4. Verfahren nach Anspruch 3, wobei eine erste Stufe umfasst:
Anzeigen von erfolgreichem Herunterladen der Anwendung,
Verifizieren eines Patienten-Passworts,
Aufnehmen eines Patienten-Testvideos, Verschlüsseln des Patienten-Testvideos,
Einrichten einer sicheren Verbindung über das Netzwerk zu der sicheren Patienten-Datenbank, und
Hochladen eines verschlüsselten Patienten-Testvideos in die sichere Patienten-Datenbank, oder
wobei eine erste Stufe umfasst
Empfangen einer Eingabe des medizinischen Dienstleisters zum Verifizieren einer Einhaltung des Medikamenten-Verabreichungsplans durch den Patienten über eine vorgegebene Anzahl von Verabreichungen, und
Anzeigen eines Fortschreitens des Patienten zu einer nachfolgenden Stufe.

5. Verfahren nach Anspruch 3, wobei die Vorgänge Weiterleiten eines Patienten über mehrere Stufen einschließen, die hohe, mittlere und niedrige Werte von Interventions-Intensität des medizinischen Dienstleisters erfordern.

6. Verfahren nach Anspruch 1, das des Weiteren umfasst:
Verschlüsseln der Videodaten-Datei,
Einrichten einer sicheren Verbindung über ein Netzwerk zu einer sicheren Patienten-Datenbank,
Hochladen einer verschlüsselten Videodaten-Datei in die sichere Patienten-Datenbank.

7. System zum Sicherstellen von Einhaltung von Medikation aus der Ferne über ein Netzwerk, das in Verbindung mit einer Computervorrichtung eines Patienten und einer Computervorrichtung eines medizinischen Dienstleisters steht, wobei es umfasst:
wenigstens eine sichere Patienten-Datenbank, in der gespeichert sind:
verschlüsselte Patienten-Profile, die Medikations-Beschreibungen, Medikamenten-Dosierungen und Verabreichungspläne einschließen,
verschlüsselte Patienten-Videos von Patienten, die Medikamente einnehmen;
ein nichtflüchtiges, computerlesbares Speichermedium für ein Patienten-Programm, das Befehle enthält, die so konfiguriert sind, dass sie einen Prozessor in der Patienten-Computervorrichtung veranlassen, die Vorgänge durchzuführen, die einschließen:
Verifizieren eines Patienten-Passworts,
Aufzeichnen einer oder mehrerer Videodaten-Datei/en, einschließlich eines Patienten-Videos, das den Patienten zeigt, der ein Medikament einnimmt,
Verschlüsseln des Patienten-Videos,
Einrichten einer sicheren Verbindung über das Netzwerk zu der sicheren Patienten-Datenbank,
Hochladen eines verschlüsselten Patienten-Videos in die sichere Patienten-Datenbank;
ein nichtflüchtiges computerlesbares Speichermedium für ein Programm eines medizinischen Dienstleisters, das Befehle enthält, die so konfiguriert sind, dass sie einen Prozessor in der Computervorrichtung des medizinischen Dienstleisters veranlassen, Vorgänge durchzuführen, die einschließen:
Verifizieren eines Passworts des medizinischen Dienstleisters,
Einrichten einer sicheren Verbindung über das Netzwerk zu der sicheren Patienten-Datenbank,
Anzeigen von Informationen bezüglich mehrerer Patienten in einer Triage-Reihenfolge,
Zugreifen auf ein verschlüsseltes Patienten-Video in der sicheren Patienten-Datenbank in Reaktion auf eine Auswahl durch einen medizinischen Dienstleister, Entschlüsseln des verschlüsselten Patienten-Videos,
Empfangen von Zustimmungs-Daten, die Annahme oder Ablehnung der einen oder mehreren Videodaten-Dateien als Beleg dafür, dass der Patient eine korrekte Dosierung des Medikaments einnimmt, wie sie in einem Medikamenten-Verabreichungsplan bestimmt ist, durch den medizinischen Dienstleister entsprechen;
ein nicht-übertragbares computerlesbares Speichermedium für ein Systemprogramm, das Befehle enthält, die so konfiguriert sind, dass sie einen Prozessor veranlassen, Vorgänge durchzuführen, die einschließen:
Berechnen eines Wertes für Einhaltung des Medikationsplans auf Basis des Medikamenten-Verabreichungsplans und der Zustimmungs-Daten; sowie
Modifizieren einer Interventions-Intensität des medizinischen Dienstleisters auf Basis des Wertes für Einhaltung des Medikationsplans,
wobei auf die Computervorrichtung des Patienten eine Anwendung für sicheren Zugang mit einem Passwort und Verschlüsselungsmöglichkeit geladen wird, und bevor dem Patienten erlaubt wird, Patienten-Informationen einzusehen, Berechtigungen des Patienten überprüft werden, um festzustellen, ob dem Patienten gestattet wird, auf ein betreffendes Objekt zuzugreifen, und wobei der Patient nicht auf die aufgezeichneten Videodaten-Dateien auf der Computervorrichtung des Patienten unter Verwendung einer standardgemäßen System-Medienbibliothek zugreifen kann, während sie auf der Computervorrichtung des Patienten gespeichert sind; und
wobei die aufgezeichneten Videodaten-Dateien verschlüsselt werden, um Zugriff unbefugter Betrachter unter Verwendung von Verzeichnisverwaltungs-Software zu verhindern.

8. System nach Anspruch 7, wobei das nichtflüchtige computerlesbare Speichermedium für das Systemprogramm Teil des nichtflüchtigen computerlesbaren Speichermediums für das Programm des medizinischen Dienstleisters ist.

9. System nach Anspruch 7, wobei die Patienten-Videos jeweils einen Zeit-und-Datums-Stempel einer Zeit und eines Datum der Aufzeichnung enthalten, und/oder wobei die Interventions-Intensität eine Zeitspanne nach Empfang des Patienten-Videos an der sicheren Patienten-Datenbank zum Einsehen des Patienten-Videos für den medizinischen Dienstleister umfasst.

10. System nach Anspruch 7, wobei Modifizieren einer Interventions-Intensität des medizinischen Dienstleisters auf Basis des Wertes für Einhaltung des Medikationsplans umfasst, dass der Patient zu einer nachfolgenden Überwachungsstufe weitergeführt wird, wobei eine abschließende Überwachungsstufe eine geringere Interventions-Intensität erfordert.

11. System nach Anspruch 10, wobei eine erste Stufe Befehle in dem nichtflüchtigen, computerlesbaren Speichermedium für das Patienten-Programm umfasst, die so konfiguriert sind, dass sie den Prozessor in der Computervorrichtung des Patienten veranlassen, Vorgänge durchzuführen, die einschließen:
Anzeigen von erfolgreichem Herunterladen des Patienten-Programms,
Verifizieren eines Patienten-Passworts,
Aufnehmen eines Patienten-Testvideos,
Verschlüsseln des Patienten-Testvideos,
Einrichten einer sicheren Verbindung über das Netzwerk zu der sicheren Patienten-Datenbank,
Hochladen eines verschlüsselten Patienten-Testvideos in die sichere Patienten-Datenbank.

12. System nach Anspruch 10, wobei eine erste Stufe Befehle in dem nichtflüchtigen, computerlesbaren Speichermedium für das Programm des medizinischen Dienstleisters umfasst, die so konfiguriert sind, dass sie den Prozessor in der Computervorrichtung des medizinischen Dienstleisters veranlassen, Vorgänge durchzuführen, die einschließen:
Empfangen von Eingabe des medizinischen Dienstleisters zum Verifizieren einer Einhaltung des Medikamenten-Verabreichungsplans durch den Patienten über eine vorgegebene Anzahl von Verabreichungen,
Anzeigen eines Fortschreitens des Patienten zu einer nachfolgenden Stufe.

13. System nach Anspruch 10, wobei die Vorgänge Weiterleiten eines Patienten über mehrere Stufen einschließen, die hohe, mittlere und niedrige Werte von Interventions-Intensität des medizinischen Dienstleisters erfordern.

14. System nach Anspruch 7, wobei das Netzwerk das Internet umfasst und die sichere Verbindung über das Netzwerk einen SSL-Tunnel umfasst.

15. System nach Anspruch 11,
wobei das Patienten-Video einen Zeitstempel einer Aufnahmezeit enthält,
Einrichten der jeweiligen sicheren Verbindung Einrichten einer sicheren SSL-Tunnelverbindung über das Netzwerk zu der sicheren Patienten-Datenbank umfasst,
wobei die Interventions-Intensität eine Zeitspanne nach Empfang des Patienten-Videos an der sicheren Patienten-Datenbank zum Einsehen des Patienten-Videos für den medizinischen Dienstleister umfasst, das Modifizieren umfasst, dass der Patient zu einer nachfolgenden Überwachungsstufe weitergeführt wird, und durch eine abschließende Überwachungsstufe die Eingriffsintensität modifiziert wird.

## Revendications

1. Procédé mis en œuvre sur un ordinateur comprenant :
une réception, par un processeur, de données de dose correspondant à une posologie de médicament pour un médicament prescrit à un patient ;
une réception, par le processeur depuis un dispositif mobile distant d'un patient, d'un ou plusieurs fichiers de données vidéo enregistrés sur le dispositif mobile du patient, incluant une vidéo du patient s'administrant le médicament à des intervalles définis par la posologie de médicament ;
une réception d'une demande provenant d'un fournisseur médical d'accès l'un ou plusieurs fichiers de données vidéo ;
une fourniture au fournisseur médical d'un accès à l'un ou plusieurs fichiers de données vidéo ;
une réception de données d'approbation correspondant à une acceptation ou un rejet par le fournisseur médical de l'un ou plusieurs fichiers de données vidéo comme preuve de la prise par le patient d'une dose correcte du médicament telle que définie dans la posologie de médicament ;
un calcul d'une valeur d'adhésion à un régime médicamenteux selon la posologie de médicament et les données d'approbation ;
dans lequel le fournisseur médical est en mesure de passer en revue plusieurs fichiers de données vidéo simultanément,
dans lequel le dispositif mobile de patient dispose d'une application chargée pour un accès sécurisé avec un mot de passe et une capacité de chiffrement dans lequel, avant de permettre au patient de consulter des informations de patient, des autorisations de patient sont vérifiées pour déterminer si le patient est autorisé ou non à accéder à un objet en question, et dans lequel le patient ne peut pas accéder aux fichiers de données vidéo enregistrés sur le dispositif mobile de patient en utilisant une bibliothèque de médias de système standard lorsqu'ils sont stockés sur le dispositif mobile de patient ; et
dans lequel les fichiers de données vidéo enregistrés sont chiffrés pour refuser un accès à des observateurs non autorisés au moyen d'un logiciel de gestion de répertoires.

2. Le procédé de la revendication 1, dans lequel le calcul d'une valeur d'adhésion à un régime médicamenteux est effectué par un programme de système séparé d'un programme de fournisseur médical sur un dispositif de fournisseur médical et/ou dans lequel les fichiers de données vidéo incluent chacun un horodatage d'heure et de date d'une heure et d'une date d'enregistrement.

3. Le procédé de la revendication 1 comprenant en outre :
une modification d'une intensité d'intervention de fournisseur médical selon la valeur d'adhésion à un régime médicamenteux, dans lequel l'intensité d'intervention comprend une quantité de temps après réception de la vidéo de patient au niveau de la base de données de patients sécurisée pour que le fournisseur médical passe en revue la vidéo de patient, en particulier, dans lequel une modification d'une intensité d'intervention de fournisseur médical selon la valeur d'adhésion à un régime médicamenteux comprend une progression du patient vers une phase de surveillance suivante, dans lequel une phase de surveillance finale nécessite une intensité moindre d'intervention.

4. Le procédé de la revendication 3, dans lequel une première phase comprend :
une indication d'un téléchargement réussi de l'application,
une vérification d'un mot de passe de patient,
une capture d'une vidéo de test de patient,
un chiffrement de la vidéo de test de patient,
un établissement d'une connexion sécurisée sur le réseau vers la base de données de patients sécurisée, et
un téléchargement d'une vidéo de test de patient chiffrée vers la base de données de patients sécurisée, ou
dans lequel une première phase comprend
une réception d'une entrée de fournisseur médical vérifiant une adhésion par le patient à la posologie de médicament pour un nombre prédéterminé de doses, et
une indication d'une avancée du patient vers une phase suivante.

5. Le procédé de la revendication 3, dans lequel les opérations incluent une avancée du patient sur plusieurs phases nécessitant des valeurs élevée, moyenne et faible d'intensité d'intervention de fournisseur médical.

6. Le procédé de la revendication 1 comprenant en outre :
un chiffrement du fichier de données vidéo,
un établissement d'une connexion sécurisée sur le réseau vers une base de données de patients sécurisée,
un téléchargement d'un fichier de données vidéo chiffré vers la base de données de patients sécurisée.

7. Système pour sécuriser une adhésion médicamenteuse à distance sur un réseau en communication avec un dispositif informatique de patient et un dispositif informatique de fournisseur médical, comprenant :
au moins une base de données de patient sécurisée stockant
des profils de patient chiffrés incluant des descriptions de médicament, des doses de médicament et des posologies,
des vidéos de patient chiffrées de patients prenant des médicaments ;
un support de stockage non transitoire lisible par un ordinateur de programme de patient contenant des instructions configurées pour faire exécuter à un processeur sur le dispositif informatique de patient des opérations incluant
une vérification d'un mot de passe de patient,
un enregistrement d'un ou plusieurs fichiers de données vidéo incluant une vidéo de patient montrant le patient prenant un médicament,
un chiffrement de la vidéo de patient,
un établissement d'une connexion sécurisée sur le réseau vers la base de données de patients sécurisée,
un téléchargement d'une vidéo de de patient chiffrée vers la base de données de patients sécurisée ;
un support de stockage non transitoire lisible par un ordinateur de programme de fournisseur médical contenant des instructions configurées pour faire exécuter à un processeur sur le dispositif informatique de fournisseur médical des opérations incluant
une vérification d'un mot de passe de fournisseur médical,
un établissement d'une connexion sécurisée sur le réseau vers la base de données de patients sécurisée,
un affichage d'informations relatives à plusieurs patients dans un ordre trié,
un accès à une vidéo de patient chiffrée dans la base de données de patients sécurisée en réponse à une sélection par un fournisseur médical,
un déchiffrement de la vidéo de patient chiffrée,
une réception de données d'approbation correspondant à une acceptation ou un rejet par le fournisseur médical de l'un ou plusieurs fichiers de données vidéo comme preuve de la prise par le patient d'une dose correcte du médicament telle que définie dans une posologie de médicament ;
un support de stockage non transitoire lisible par un ordinateur de programme de système contenant des instructions configurées pour faire exécuter à un processeur des opérations incluant
un calcul d'une valeur d'adhésion à un régime médicamenteux selon la posologie de médicament et les données d'approbation ; et
une modification d'une intensité d'intervention de fournisseur médical selon la valeur d'adhésion à un régime médicamenteux,
dans lequel le dispositif informatique de patient dispose d'une application chargée pour un accès sécurisé avec un mot de passe et une capacité de chiffrement, dans lequel, avant de permettre au patient de consulter des informations de patient, les autorisations de patient sont vérifiées pour déterminer si le patient est autorisé ou non à accéder à un objet en question, et dans lequel le patient ne peut pas accéder aux fichiers de données vidéo enregistrés sur le dispositif informatique de patient en utilisant une bibliothèque de médias de système standard lorsqu'ils sont stockés sur le dispositif informatique de patient ; et
dans lequel les fichiers de données vidéo enregistrés sont chiffrés pour refuser un accès à des observateurs non autorisés au moyen d'un logiciel de gestion de répertoires.

8. Le système de la revendication 7 dans lequel le support de stockage non transitoire lisible par un ordinateur de programme de système fait partie du support de stockage non transitoire lisible par un ordinateur de fournisseur médical.

9. Le système de la revendication 7 dans lequel les vidéos de patient incluent chacune un horodatage d'heure et de date d'une heure et d'une date d'enregistrement, et/ou dans lequel l'intensité d'intervention comprend une quantité de temps après réception de la vidéo de patient au niveau de la base de données de patients sécurisée pour que le fournisseur médical passe en revue la vidéo de patient.

10. Le système de la revendication 7 dans lequel une modification d'une intensité d'intervention de fournisseur médical selon la valeur d'adhésion à un régime médicamenteux comprend une progression du patient vers une phase de surveillance suivante, dans lequel une phase de surveillance finale nécessite une intensité moindre d'intervention.

11. Le système de la revendication 10 dans lequel une première phase comprend des instructions sur le support de stockage non transitoire lisible par un ordinateur de programme de patient configurées pour faire exécuter au processeur sur le dispositif informatique de patient des opérations incluant
une indication d'un téléchargement réussi du programme de patient,
une vérification d'un mot de passe de patient,
une capture d'une vidéo de test de patient,
un chiffrement de la vidéo de test de patient,
un établissement d'une connexion sécurisée sur le réseau vers la base de données de patients sécurisée,
un téléchargement d'une vidéo de test de patient chiffrée vers la base de données de patients sécurisée.

12. Le système de la revendication 10 dans lequel une première phase comprend des instructions sur le support de stockage non transitoire lisible par un ordinateur de programme de fournisseur médical configurées pour faire exécuter au processeur sur le dispositif informatique de fournisseur médical des opérations incluant
une réception d'une entrée de fournisseur médical vérifiant une adhésion par le patient à la posologie de médicament pour un nombre prédéterminé de doses,
une indication d'une avancée du patient vers une phase suivante.

13. Le système de la revendication 10 dans lequel les opérations incluent une avancée du patient sur plusieurs phases nécessitant des valeurs élevée, moyenne et faible d'intensité d'intervention de fournisseur médical.

14. Le système de la revendication 7 dans lequel le réseau comprend Internet et la connexion sécurisée sur le réseau comprend un tunnel SSL.

15. Le système selon la revendication 11,
dans lequel la vidéo de patient inclut un horodatage d'heure d'une heure d'enregistrement,
dans lequel un établissement de la connexion sécurisée respective comprend un établissement d'une connexion en tunnel SSL sécurisée sur le réseau vers la base de données de patients sécurisée,
dans lequel l'intensité d'intervention comprend une quantité de temps après réception de la vidéo de patient au niveau de la base de données de patients sécurisée pour que le fournisseur médical passe en revue la vidéo de patient, dans lequel la modification comprend une avancée du patient vers une phase de surveillance suivante, dans lequel une phase de surveillance finale modifie l'intensité d'intervention.
